(19) Europäisches Patentamt
European Patent Office
Office européen des brevets

(11) EP 2 007 417 B1

(12) **EUROPEAN PATENT SPECIFICATION**

(45) Date of publication and mention of the grant of the patent:
**20.10.2010 Bulletin 2010/42**

(21) Application number: **07727577.4**

(22) Date of filing: **30.03.2007**

(51) Int Cl.:
***A61K 38/48*** *(2006.01)* ***C12N 9/64*** *(2006.01)*
***C07K 14/705*** *(2006.01)* ***C07K 14/745*** *(2006.01)*

(86) International application number:
**PCT/EP2007/053105**

(87) International publication number:
**WO 2007/115953 (18.10.2007 Gazette 2007/42)**

(54) **COVALENT FACTOR VII-TISSUE FACTOR COMPLEX**

KOVALENTER FAKTOR VII-GEWEBEFAKTOR-KOMPLEX

COMPLEXE COVALENT DU FACTEUR VII ET DU FACTEUR TISSULAIRE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **07.04.2006 EP 06112358**

(43) Date of publication of application:
**31.12.2008 Bulletin 2009/01**

(73) Proprietor: **Novo Nordisk Health Care AG**
**8050 Zürich (CH)**

(72) Inventors:
- **ØSTERGAARD, Henrik**
  **3650 Ølstykke (DK)**
- **OLSEN, Ole, Hvilsted**
  **2700 Brønshøj (DK)**
- **PETERSEN, Anders Klarskov**
  **2850 Nærum (DK)**
- **STENNICKE, Henning Ralf**
  **2980 Kokkedal (DK)**

(74) Representative: **Nilsson, Karin Norvin**
**Novo Nordisk A/S**
**Corporate Patents**
**Novo Allé**
**2880 Bagsværd (DK)**

(56) References cited:
**WO-A-01/58935 WO-A-97/20939**
**WO-A-03/092602 WO-A-2004/006962**

- **MIYATA TOSHIYUKI ET AL: "Chemical cross-linking of activated coagulation factor VII with soluble tissue factor: Calcium ions are not essential for full amidolytic activity of the factor VIIa-tissue factor complex after complex formation" JOURNAL OF BIOCHEMISTRY (TOKYO), vol. 117, no. 4, 1995, pages 836-844, XP009071371 ISSN: 0021-924X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 007 417 B1

**Description**

FIELD OF THE INVENTION

The present invention relates to novel covalent complexes of a Factor VII polypeptide and a Tissue Factor polypeptide, in particular to such complexes which are functionally active and which have an enhanced proteolytic activity towards Factor X compared to the corresponding free Factor VII polypeptide. Such complexes are believed to particularly useful in the treatment of bleeding disorders.

BACKGROUND OF THE INVENTION

Factor VIIa polypeptides with enhanced biological activity are desirable molecules for the treatment of uncontrolled bleedings that are partially or completely refractory to conventional recombinant Factor VIIa therapy due to suboptimal potency of recombinant Factor VIIa. Current approaches to enhance potency reflect the mechanism of action of recombinant Factor VIIa which appears to be direct Factor X activation on the activated platelet independent of Tissue Factor.

Tissue Factor is a 263 amino acid integral membrane glycoprotein receptor residing on the cells of the vascular adventitia. It consists of an extracellular part folded into two compact fibronectin type III-like domains (1-219) each stabilized by a single disulfide bond, a transmembrane segment (220-242), and a short cytoplasmic tail (243-263). It serves as the key inhibitor of coagulation by forming a tight $Ca^{2+}$-dependent complex with Factor VII which is captured from circulation upon vascular injury. Tissue Factor also greatly enhances the proteolytic activity of Factor VIIa towards its physiologic substrates Factor IX and Factor X by providing a scaffold for optimal macromolecular exosite interactions and by inducing conformational changes in the protease domain of Factor VIIa resulting in correct definition of the active-site region. The conformational activation of FVIIa by TF which results from direct protein-protein interaction can be recapitulated *in vitro* by supplying Factor VIIa with the soluble ectodomain of Tissue Factor (sTF).

Miyata et al., Biochemistry, 36, 1997, 5120-5127, disclose a covalent complex of recombinant Factor VIIa and soluble Tissue Factor being chemically cross-linked by means of a homo-bifunctional amine-specific reagent. However the cross-linked complex did not exhibit proteolytic activity towards Factor X.

BRIEF DESCRIPTION OF THE INVENTION

The present invention relates to novel covalent complexes of a Factor VII polypeptide and a Tissue Factor polypeptide. The complexes exhibit enhanced proteolytic activity towards the macromolecular substrate Factor X relative to the corresponding free Factor VII polypeptide.

More particular, the present invention relates to a covalent complex of a Factor VII polypeptide and a Tissue Factor polypeptide, wherein the Factor VII polypeptide and the Tissue Factor polypeptide are covalently linked by means of one or more designated links between amino acid side chains of sets of (i) an amino acid of the Factor VII polypeptide and (ii) an amino acid of the Tissue Factor polypeptide.

Moreover, the present invention also relates to a covalent complex of a Factor VIIa polypeptide and a Tissue Factor polypeptide, wherein the Factor VIIa polypeptide and the Tissue Factor polypeptide are covalently linked by means of one or more links between amino acid side chains of sets of (i) an amino acid of the Factor VIIa polypeptide and (ii) an amino acid of the Tissue Factor polypeptide, wherein the proteolytic activity of the Factor VIIa polypeptide is at least 2-fold, such as at least 50-fold, e.g. at least 100-fold, such as at least 200-fold, or at least 250-fold, of that of the free native (wild-type) factor VIIa, as determined by the *in vitro* proteolysis assay defined herein.

The present invention also relates to a method for production of a complex of a Factor VII polypeptide and a Tissue Factor polypeptide as defined herein, the method comprising a) transfecting a cell with (i) an expression vector comprising a nucleic acid molecule encoding the Factor VII polypeptide and expression control regions operatively linked to thereto and (ii) and an expression vector comprising a nucleic acid molecule encoding the Tissue Factor polypeptide and expression control regions operatively linked to thereto, b) culturing the transfected cell under conditions for expression of Factor VII polypeptide and Tissue Factor polypeptide, and c) isolating the expressed complex by suitable means.

BRIEF DESCRIPTION OF THE DRAWINGS

Figure 1 shows a model of the covalent complex of Factor VIIa and soluble Tissue Factor containing an interface-spanning disulfide between V207C in soluble Tissue Factor and F40C in Factor VIIa. The $C_o$-distance from V207C or F40C to native (wild-type) cysteine residues across the interface is significantly greater than 7 Å which precludes

the formation of unintentional disulfide pairings between the two molecules.

Figure 2 shows a Coomassie-stained SDS-polyacrylamide gel of native (wild-type) factor VIIa (lane A) and the purified factor VIIa F40C-sTF(1-219) V207C (lane B) complex under non-reducing and reducing conditions, respectively. HC and LC represent the heavy and light chains of factor VIIa.

Figure 3 shows an immunoblot of wild-type factor VIIa (lane A), conditioned medium from transient co-expression of factor VII Q64C and sTF(1-219) G109C (lane B), and purified factor VIIa F40C-sTF(1-219) V207C complex (lane C).

## DETAILED DESCRIPTION OF THE INVENTION

The present invention, i.a., relates to a covalent complex of a Factor VII polypeptide and a Tissue Factor polypeptide, wherein the Factor VII polypeptide and the Tissue Factor polypeptide are covalently linked by means of one or more designated links between amino acid side chains of sets of (i) an amino acid of the Factor VII polypeptide and (ii) an amino acid of the Tissue Factor polypeptide.

In the present context, the term "covalent complex" refers to two molecules (here: a Factor VII polypeptide and a Tissue Factor polypeptide) associated by at least one covalent link or bond and a plurality of non-covalent interactions, e.g. hydrogen bonds, hydrophobic interactions, etc. Hence, the Factor VII polypeptide and the Tissue Factor polypeptide are covalently linked in this manner.

The two polypeptides are linked by means of one or more designated links between amino acid side chains of sets of (i) an amino acid of the Factor VII polypeptide and (ii) an amino acid of the Tissue Factor polypeptide.

When used herein, the term "Factor VII polypeptide" encompasses wild-type Factor VII (i.e. a polypeptide having the amino acid sequence disclosed in U.S. Patent No. 4,784,950), as well as variants of Factor VII, Factor VII derivatives and Factor VII conjugates exhibiting substantially the same or improved biological activity relative to wild-type Factor VII. The term "Factor VII" is intended to encompass Factor VII polypeptides in their uncleaved (zymogen) form, as well as those that have been proteolytically processed to yield their respective bioactive forms, which may be designated Factor VIIa. Typically, Factor VII is cleaved between residues 152 and 153 to yield Factor VIIa. The term "Factor VII polypeptide" also encompasses polypeptides, including variants, in which the Factor VIIa biological activity has been substantially modified or somewhat reduced relative to the activity of wild-type Factor VIIa. These polypeptides include, without limitation, Factor VII or Factor VIIa into which specific amino acid sequence alterations have been introduced that modify or disrupt the bioactivity of the polypeptide.

The biological activity of Factor VIIa in blood clotting derives from its ability to (i) bind to Tissue Factor (TF) and (ii) catalyze the proteolytic cleavage of Factor IX or Factor X to produce activated Factor IX or X (Factor IXa or Xa, respectively).

In the present context, the term "functionally active Factor VII polypeptide" refers to "Factor VII polypeptide" that have been proteolytically processed to yield their respective bioactive forms, and which frequently are referred to as "Factor VIIa polypeptides".

In some important embodiments, the Factor VII polypeptide is functionally active.

As used herein, "wild type human FVIIa" is a polypeptide having the amino acid sequence disclosed in U.S. Patent No. 4,784,950.

The term "Factor VII derivative" as used herein, is intended to designate a FVII polypeptide exhibiting substantially the same or improved biological activity relative to wild-type Factor VII, in which one or more of the amino acids of the parent peptide have been genetically and/or chemically and/or enzymatically modified, e.g. by alkylation, glycosylation, PEGylation, acylation, ester formation or amide formation or the like. This includes but is not limited to PEGylated human Factor VIIa, cysteine-PEGylated human Factor VIIa and variants thereof. Non-limiting examples of Factor VII derivatives includes GlycoPegylated FVII derivatives as disclosed in WO 03/31464 and US Patent applications US 20040043446, US 20040063911, US 20040142856, US 20040137557, and US 20040132640 (Neose Technologies, Inc.); FVII conjugates as disclosed in WO 01/04287, US patent application 20030165996, WO 01/58935, WO 03/93465 (Maxygen ApS) and WO 02/02764, US patent application 20030211094 (University of Minnesota).

The term "improved biological activity" refers to FVII polypeptides with i) substantially the same or increased proteolytic activity compared to recombinant wild type human Factor VIIa or ii) to FVII polypeptides with substantially the same or increased TF binding activity compared to recombinant wild type human Factor VIIa or iii) to FVII polypeptides with substantially the same or increased half life in blood plasma compared to recombinant wild type human Factor VIIa. The term "PEGylated human Factor VIIa" means human Factor VIIa, having a PEG molecule conjugated to a human Factor VIIa polypeptide. It is to be understood, that the PEG molecule may be attached to any part of the Factor VIIa polypeptide including any amino acid residue or carbohydrate moiety of the Factor VIIa polypeptide. The term "cysteine-PEGylated human Factor VIIa" means Factor VIIa having a PEG molecule conjugated to a sulfhydryl group of a cysteine introduced in human Factor VIIa.

Non-limiting examples of additional substitutions of amino acids in the Factor VII polypeptide providing Factor VII polypeptides with substantially the same or increased proteolytic activity compared to recombinant wild type human Factor VIIa include S52A, S60A ( Lino et al., Arch. Biochem. Biophys. 352: 182-192, 1998); substitutions providing FVIIa polypeptides exhibiting increased proteolytic stability as disclosed in U.S. Patent No. 5,580,560; substitutions providing FVIIa polypeptides that has been proteolytically cleaved between residues 290 and 291 or between residues 315 and 316 (Mollerup et al., Biotechnol. Bioeng. 48:501-505, 1995); oxidized forms of Factor VIIa (Kornfelt et al., Arch. Biochem. Biophys. 363:43-54, 1999); substitutions in FVIIa polypeptides as disclosed in WO 02/077218; and substitutions in FVIIa polypeptides, which exhibit increased pro-teolytic stability as disclosed in WO 02/38162 (Scripps Research Institute); substitutions in FVIIa polypeptides, which provides polypeptides having a modified Gla-domain and exhibiting an enhanced membrane binding as disclosed in WO 99/20767, US patents US 6017882 and US 6747003, US patent application 20030100506 (University of Minnesota) and WO 00/66753, US patent applications US 20010018414, US 2004220106, and US 200131005, US patents US 6762286 and US 6693075 (University of Minnesota); and substitutions in FVIIa polypeptides as disclosed in WO 01/58935, US patent US 6806063, US patent application 20030096338 (Maxygen ApS), WO 03/93465 (Maxygen ApS), WO 04/029091 (Maxygen ApS), WO 04/083361 (Maxygen ApS), and WO 04/111242 (Maxygen ApS), as well as in WO 04/108763 (Canadian Blood Services).

Non-limiting examples of further substitutions in FVIIa polypeptides pro-viding FVII polypeptides having increased biological activity compared to wild-type FVIIa include substitutions as disclosed in WO 01/83725, WO 02/22776, WO 02/077218, WO 03/027147, WO 04/029090, WO 03/027147; WO 02/38162 (Scripps Research Institute); and FVIIa polypeptides with enhanced activity as dis-closed in JP 2001061479 (Chemo-Sero-Therapeutic Res Inst.).

Examples of further substitutions in the FVIIa polypeptides of the present invention include, without limitation, L305V, L305V/M306D/D309S, L305I, L305T, F374P, V158T/M298Q, V158D/E296V/M298Q, K337A, M298Q, V158D/M298Q, L305V/K337A, V158D/E296V/M298Q/L305V, V158D/E296V/M298Q/K337A, V158D/E296V/M298Q/L305V/K337A, K157A, E296V, E296V/M298Q, V158D/E296V, V158D/M298K, and S336G, L305V/K337A, L305V/V158D, L305V/E296V, L305V/M298Q, L305V/V158T, L305V/K337A/V158T, L305V/K337A/M298Q, L305V/K337A/E296V, L305V/K337A/V158D, L305V/V158D/M298Q, L305V/V158D/E296V, L305V/V158T/M298Q, L305V/V158T/E296V, L305V/E296V/M298Q, L305V/V158D/E296V/M298Q, L305V/V158T/E296V/M298Q, L305V/V158T/K337A/M298Q, L305V/V158T/E296V/K337A, L305V/V158D/K337A/M298Q, L305V/V158D/E296V/K337A, L305V/V158D/E296V/M298Q/K337A, L305V/V158T/E296V/M298Q/K337A, S314E/K316H, S314E/K316Q, S314E/L305V, S314E/K337A, S314E/V158D, S314E/E296V, S314E/M298Q, S314E/V158T, K316H/L305V, K316H/K337A, K316H/V158D, K316H/E296V, K316H/M298Q, K316H/V158T, K316Q/L305V, K316Q/K337A, K316Q/V158D, K316Q/E296V, K316Q/M298Q, K316Q/V158T, S314E/L305V/K337A, S314E/L305V/V158D, S314E/L305V/E296V, S314E/L305V/M298Q, S314E/L305V/V158T, S314E/L305V/K337A/V158T, S314E/L305V/K337A/M298Q, S314E/L305V/K337A/E296V, S314E/L305V/K337A/V158D, S314E/L305V/V158D/M298Q, S314E/L305V/V158D/E296V, S314E/L305V/V158T/M298Q, S314E/L305V/V158T/E296V, S314E/L305V/E296V/M298Q, S314E/L305V/V158D/E296V/M298Q, S314E/L305V/V158T/E296V/M298Q, S314E/L305V/V158T/K337A/M298Q, S314E/L305V/V158T/E296V/K337A, S314E/L305V/V158D/K337A/M298Q, S314E/L305V/V158D/E296V/K337A, S314E/L305V/V158D/E296V/M298Q/K337A, S314E/L305V/V158T/E296V/M298Q/K337A, K316H/L305V/K337A, K316H/L305V/V158D, K316H/L305V/E296V, K316H/L305V/M298Q, K316H/L305V/V158T, K316H/L305V/K337A/V158T, K316H/L305V/K337A/M298Q, K316H/L305V/K337A/E296V, K316H/L305V/K337A/V158D, K316H/L305V/V158D/M298Q, K316H/L305V/V158D/E296V, K316H/L305V/V158T/M298Q, K316H/L305V/V158T/E296V, K316H/L305V/E296V/M298Q, K316H/L305V/V158D/E296V/M298Q, K316H/L305V/V158T/E296V/M298Q, K316H/L305V/V158T/K337A/M298Q, K316H/L305V/V158T/E296V/K337A, K316H/L305V/V158D/K337A/M298Q, K316H/L305V/V158D/E296V/K337A, K316H/L305V/V158D/E296V/M298Q/K337A, K316H/L305V/V158T/E296V/M298Q/K337A, K316Q/L305V/K337A, K316Q/L305V/V158D, K316Q/L305V/E296V, K316Q/L30SV/M298Q, K316Q/L305V/V158T, K316Q/L305V/K337A/V158T, K316Q/L305V/K337A/M298Q, K316Q/L305V/K337A/E296V, K316Q/L305V/K337A/V158D, K316Q/L305V/V158D/M298Q, K316Q/L305V/V158D/E296V, K316Q/L305V/V158T/M298Q, K316Q/L305V/V158T/E296V, K316Q/L305V/E296V/M298Q, K316Q/L305V/V158D/E296V/M298Q, K316Q/L305V/V158T/E296V/M298Q, K316Q/L305V/V158T/K337A/M 298Q, K316Q/L305V/V158T/E296V/K337A, K316Q/L305V/V158D/K337A/M298Q, K316Q/L305V/V158D/E296V/K337A, K316Q/L305V/V158D/E296V/M298Q/K337A, K316Q/L305V/V158T/E296V/M298Q/K337A, F374Y/K337A, F374Y/V158D, F374Y/E296V, F374Y/M298Q, F374Y/V158T, F374Y/S314E, F374Y/L305V, F374Y/L305V/K337A, F374Y/L305V/V158D, F374Y/L305V/E296V, F374Y/L305V/M298Q, F374Y/L305V/V158T, F374Y/L305V/S314E, F374Y/K337A/S314E, F374Y/K337A/V158T, F374Y/K337A/M298Q, F374Y/K337A/E296V, F374Y/K337A/V158D, F374Y/V158D/S314E, F374Y/V158D/M298Q, F374Y/V158D/E296V, F374Y/V158T/S314E, F374Y/V158T/M298Q, F374Y/V158T/E296V, F374Y/E296V/S314E, F374Y/S314E/M298Q, F374Y/E296V/M298Q, F374Y/L305V/K337A/V158D, F374Y/L305V/K337A/E296V, F374Y/L305V/K337A/M298Q, F374Y/L305V/K337A/V158T, F374Y/L305V/K337A/S314E, F374Y/L305V/V158D/E296V, F374Y/L305V/V158D/M298Q, F374Y/L305V/V158D/S314E, F374Y/L305V/E296V/M298Q, F374Y/L305V/E296V/V158T, F374Y/L305V/E296V/S314E, F374Y/L305V/M298Q/V158T, F374Y/L305V/M298Q/S314E, F374Y/L305V/V158T/S314E, F374Y/K337A/S314E/V158T, F374Y/K337A/S314E/M298Q,

F374Y/K337A/S314E/E296V, F374Y/K337A/S314E/V158D, F374Y/K337A/V158T/M298Q, F374Y/K337A/V158T/E296V, F374Y/K337A/M298Q/E296V, F374Y/K337A/M298Q/V158D, F374Y/K337A/E296V/V158D, F374Y/V158D/S314E/M298Q, F374Y/V158D/S314E/E296V, F374Y/V158D/M298Q/E296V, F374Y/V158T/S314E/E296V, F374Y/V158T/S314E/M298Q, F374Y/V158T/M298Q/E296V, F374Y/E296V/S314E/M298Q, F374Y/L305V/M298Q/K337A/S314E, F374Y/L305V/E296V/K337A/S314E, F374Y/E296V/M298Q/K337A/S314E, F374Y/L305V/E296V/M298Q/K337A, F374Y/L305V/E296V/M298Q/S314E, F374Y/V158D/E296V/M298Q/K337A, F374Y/V158D/E296V/M298Q/S314E, F374Y/L305V/V158D/K337A/S314E, F374Y/V158D/M298Q/K337A/S314E, F374Y/V158D/E296V/K337A/S314E, F374Y/L305V/V158D/E296V/M298Q, F374Y/L305V/V158D/M298Q/K337A, F374Y/L305V/V158D/E296V/K337A, F374Y/L305V/V158D/M298Q/S314E, F374Y/L305V/V158D/E296V/S314E, F374Y/V158T/E296V/M298QJK-337A, F374Y/V158T/E296V/M298Q/S314E, F374Y/L305V/V158T/K337A/S314E, F374Y/V158T/M298Q/K337A/S314E, F374Y/V158T/E296V/K337A/S314E, F374Y/L305V/V158T/E296V/M298Q, F374Y/L305V/V158T/M298Q/K337A, F374Y/L305V/V158T/E296V/K337A, F374Y/L305V/V158T/M298Q/S314E, F374Y/L305V/V158T/E296V/S314E, F374Y/E296V/M298Q/K337A/V158T/S314E, F374Y/V158D/E296V/M298Q/K337A/S314E, F374Y/L305V/V158D/E296V/M298Q/S314E, F374Y/L305V/E296V/M298Q/V158T/S314E, F374Y/L305V/E296V/M298Q/K337A/V158T, F374Y/L305V/E296V/K337A/V158T/S314E, F374Y/L305V/M298Q/K337A/V158T/S314E, F374Y/L305V/V158D/E296V/M298Q/K337A, F374Y/L305V/V158D/E296V/K337A/S314E, F374Y/L305V/V158D/M298Q/K337A/S314E, F374Y/L305V/E296V/M298Q/K337A/V158T/S314E, F374Y/L305V/V158D/E296V/M298Q/K337A/S314E, S52A, S60A; R152E, S344A, T106N, K143N/N145T, V253N, R290N/A292T, G291N, R315N/V317T, K143N/N/45T/R315N/V317T; and substitutions, additions or deletions in the amino acid sequence from 233Thr to 240Asn; substitutions, additions or deletions in the amino acid sequence from 304Arg to 329Cys; and substitutions, additions or deletions in the amino acid sequence from 153Ile to 223Arg.

The terms "variant" or "variants", as used herein, is intended to designate Factor VII having the amino acid sequence disclosed in U.S. Patent No. 4,784,950, wherein one or more amino acids of the parent protein have been substituted by another amino acid and/or wherein one or more amino acids of the parent protein have been deleted and/or wherein one or more amino acids have been inserted in protein and/or wherein one or more amino acids have been added to the parent protein. Such addition can take place either at the N-terminal end or at the C-terminal end of the parent protein or both. The "variant" or "variants" within this definition still have FVII activity in its activated form. In one embodiment a variant is 70 % identical with the amino acid sequence disclosed in U.S. Patent No. 4,784,950. In one embodiment a variant is 80 % identical with the amino acid sequence disclosed in U.S. Patent No. 4,784,950. In another embodiment a variant is 90 % identical with the amino acid sequence disclosed in U.S. Patent No. 4,784,950. In a further embodiment a variant is 95 % identical with the amino acid sequence disclosed in U.S. Patent No. 4,784,950.

In the present context, the term "Tissue Factor polypeptide" refers to a polypeptide comprising the soluble ectodomain of Tissue Factor, i.e. amino acids 1-219 (in the following referred to as sTF or sTF(1-219)), or a functional variant or truncated form thereof. Preferably, the Tissue Factor polypeptide at least comprises a fragment corresponding to the amino acid sequence 6-209 of Tissue Factor. Examples hereof are, in particular, sTF(6-209), sTF(1-209) and sTF(1-219).

The amino acid sequence of mature human Tissue Factor (1-263) is given in SEQ ID NO:2 and disclosed in Spicer et al. (1987) Proc. Natl. Acad. Sci. USA, 84, 5148-5152.

Ser$^{1}$-Gly-Thr-Thr-Asn-Thr-Val-Ala-Ala-Tyr$^{10}$-Asn-Leu-Thr-Trp-Lys-Ser-Thr-Asn-Phe-Lys$^{20}$-Thr-Ile-Leu-Glu-Trp-Glu-Pro-Lys-Pro-Val$^{30}$-Asn-Gln-Val-Tyr-Thr-Val-Gln-Ile-Ser-Thr$^{40}$-Lys-Ser-Gly-Asp-Trp-Lys-Ser-Lys-Cys-Phe$^{50}$-Tyr-Thr-Thr-Asp-Thr-Glu-Cys-Asp-Leu-Thr$^{60}$-Asp-Glu-Ile-Val-Lys-Asp-Val-Lys-Gln-Thr$^{70}$-Tyr-Leu-Ala-Arg-Val-Phe-Ser-Tyr-Pro-Ala$^{80}$-Gly-Asn-Val-Glu-Ser-Thr-Gly-Ser-Ala-Gly$^{90}$-Glu-Pro-Leu-Tyr-Glu-Asn-Ser-Pro-Glu-Phe$^{100}$-Thr-Pro-Tyr-Leu-Glu-Thr-Asn-Leu-Gly-Gln$^{110}$-Pro-Thr-Ile-Gln-Ser-Phe-Glu-Gln-Val-Gly$^{120}$-Thr-Lys-Val-Asn-Val-Thr-Val-Glu-Asp-Glu$^{130}$-Arg-Thr-Leu-Val-Arg-Arg-Asn-Asn-Thr-Phe$^{140}$-Leu-Ser-Leu-Arg-Asp-Val-Phe-Gly-Lys-Asp$^{150}$-Leu-Ile-Tyr-Thr-Leu-Tyr-Tyr-Trp-Lys-Ser$^{160}$-Ser-Ser-Ser-Gly-Lys-Lys-Thr-Ala-Lys-Thr$^{170}$-Asn-Thr-Asn-Glu-Phe-Leu-Ile-Asp-Val-Asp$^{180}$-Lys-Gly-Glu-Asn-Tyr-Cys-Phe-Ser-Val-Gln$^{190}$-Ala-Val-Ile-Pro-Ser-Arg-Thr-Val-Asn-Arg$^{200}$-Lys-Ser-Thr-Asp-Ser-Pro-Val-Glu-Cys-Met$^{210}$-Gly-Gln-Glu-Lys-Gly-Glu-Phe-Arg-Glu-Ile$^{220}$-Phe-Tyr-Ile-Ile-Gly-Ala-Val-Val-Phe-Val$^{230}$-Val-Ile-Ile-Leu-Val-Ile-Ile-Leu-Ala-Ile$^{240}$-Ser-Leu-His-Lys-Cys-Arg-Lys-Ala-Gly-Val$^{250}$-Gly-Gln-Ser-Trp-Lys-Glu-Asn-Ser-Pro-Leu$^{260}$-Asn-Val-Ser$^{263}$ (SEQ ID NO:2).

In the present context, the terms "designated link" and "designated links" are intended to mean that the Factor VII polypeptide and the Tissue Factor polypeptide are covalently linked by means of one or more sets of covalently linked amino acid side chains (possibly via a linker moiety), where the set(s) for at least 90% of the complex species are identical with respect to the position and type of at least one of the two amino acids within each of the set(s) throughout a population of complex species. Such links may either be established as direct bonds between amino acid side chains, e.g. in the form of an S-S bridge between cysteine amino acid side chains, or by means of a linker moiety linking amino acid side chains.

Preferably, at least 95%, or even at least 98%, or even virtually all, of the complex species are identical with respect to the position and type of at least one of the two amino acids within each of the set(s) throughout a population of complex species.

The number of sets of covalently linked amino acid side chains is not particularly critical, but in view of the current experimental results, it is believed that only one set is necessary. Because the links are "designated", i.e. position and type of at least one of the two amino acids within each of the set(s) is predetermined, it should be understood that the functionality and proteolytic activity of the involved polypeptides can be maintained and even enhanced, contrary to a "shotgun" approach using a common (homo)bifunctional cross-linking agents. This being said, it is believed that the number of sets of covalently linked amino acid side chains typically is in the range of 1-10, e.g. 1-5, such as 1-4, or 1-3, 1-2, or 2-4, or 2-3, or 1 or 2 link(s), in particular 1 or 2 links, preferably just one link.

Formation of one or more designated links can be accomplished either by incorporation of one or more amino acids in one or both polypeptide(s) which each are capable of reacting with an amino acid of the other polypeptide, or by addition of a cross-linking agent (most preferable a heterobifunctional cross-linking agent) which is capable of reacting with one or more specific amino acids (preferably only 1-10 amino acids).

In one important embodiment, each of the Factor VII polypeptide and the Tissue Factor polypeptide include one or more cysteine amino acids which is not designated for establishing a intramolecular disulfide bond, but which are capable of forming intermolecular disulfide bonds between the Factor VII polypeptide and the Tissue Factor polypeptide, i.e. covalent links.

In order to preserve the proteolytic activity of the Factor VII polypeptide, it is advantageous if such cysteine amino acids are located in the native interface between the Factor VII polypeptide and the Tissue Factor polypeptide in the corresponding non-covalently linked complex, e.g. as illustrated in Figure 1. If successfully designed the intramolecular disulfide bond will stabilise the complex and promote the catalytically competent conformation of Factor VIIa. The disulfide design has been performed by application of X-ray structures of the Factor VIIa / Tissue Factor complex (in particular the structure with PDB entry code 1DAN (Banner et al. (1996) Nature, 380, 41-46)). One important criterion for establishing a disulfide bond between two introduced cysteine amino acids is that the distance between their $C_\alpha$ atoms are less than 7 Ångstrom (Petersen et al. (1999) Protein Eng., 12, 535-548). Calculation of distances between $C_\alpha$ atoms in Factor VIIa and Tissue Factor in the Factor VIIa-Tissue Factor complex results in 17 potential cysteine pairs. Further evaluation of the feasibility of formation of disulfide bonds between these have been performed by modelling the disulfides (using the CHARMM package, Accelrys, SanDiego) followed by visual inspection.

Disulfides fulfilling the criteria established by Petersen et al (1999) Protein Eng., 12, 535-548 are then considered as candidates for biochemical evaluation.

In another embodiment, the Factor VII polypeptide and/or the Tissue Factor polypeptide include one or more reactive amino acids, in particular photo-reactive amino acids which, upon photo activation, are capable of forming corresponding intermolecular bonds between the Factor VII polypeptide and the Tissue Factor polypeptide, i.e. covalent links. Examples of such photo-reactive amino acids are photo-isoleucine (photo-Ile), photo-methionine (photo-Met), and photo-leucine (photo-Leu) developed by Suchanek et al: (Suchanek et al. 2005; see Scheme 1), and p-benzoyl-L-phenylalanine.

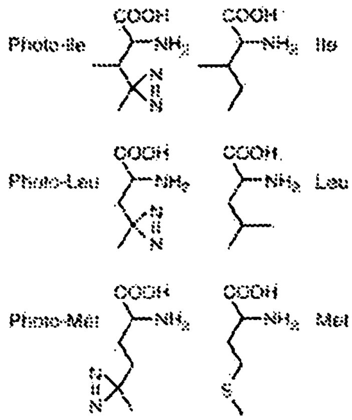

Scheme 1

Incorporation of photo-Ile, photo-Met, and photo-Leu into proteins and subsequent photo-induced cross-linking has been described by Suchanek et al. (2005) Nature methods, 2, 261-267.

Alternatively, the one or more designated links are established by means of a linker moiety between amino acid side chains of one or more sets of an amino acid of the Factor VII polypeptide and an amino acid of the Tissue Factor polypeptide. Examples of such linker moieties are those derived from a heterobifunctional reagent which is capable of reacting with a specific amino acid side chain, e.g. a cysteine side chains in one of the polypeptides, and subsequently with another amino acid side chain which is in close spatial proximity of that specific amino acid side chain when the Factor VII polypeptide and the Tissue Factor polypeptide form a non-covalently linked complex.

Examples of particularly suitable heterobifunctional reagents are those which are capable of first reacting with a cysteine amino acid side chain, and subsequently (preferably upon activation, e.g. photoactivation or rhodium catalysed) with another amino acid side chain which is in close spatial proximity. Particular examples hereof are those disclosed by Zhang et al. (Biochem. Biophys. Res. Comm., Vol. 217, 3, 1995, 1177-1184), e.g. p-azidoiodoacetanilide, p-azidophenacyl bromide and p-azidobromoacetanilide, and

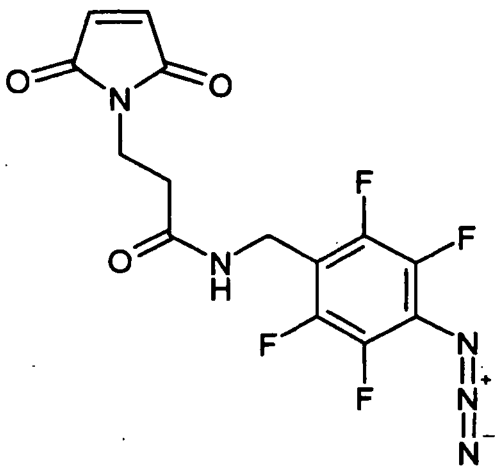

N-(4-azido-2,3,5,6-tetrafluorobenzyl)-3-maleimidylpropionamide,

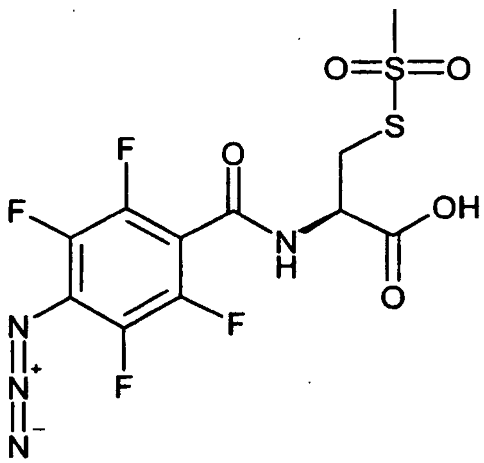

4-azido-2,3,5,6-tetrafluorobenzamidocysteine methanethiosulfonate,

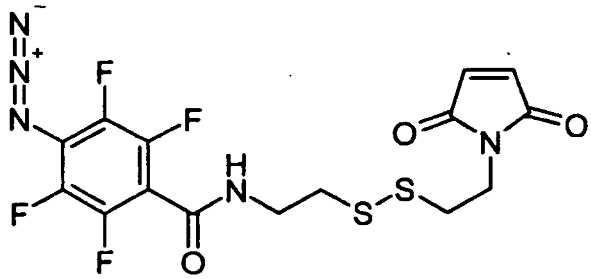

N-(2-((2-(((4-azido-2,3,5,6-tetrafluoro)benzoyl)amino)ethyl)di thio)ethyl) maleimide,

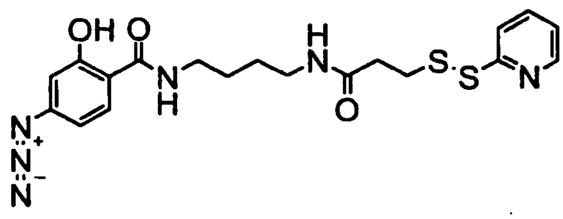

N-[4-(p-azidosalicylamido)butyl]-3'-(2'-pyridyldithio)propionamide,

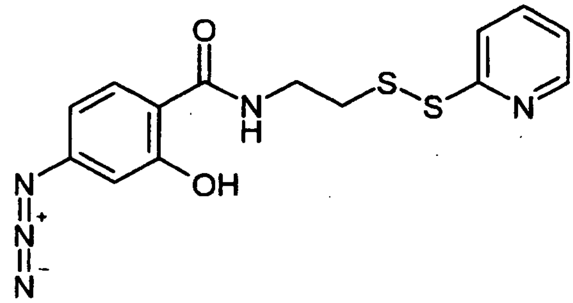

N-((2-pyridyldithio)ethyl)-4-azidosalicylamide,

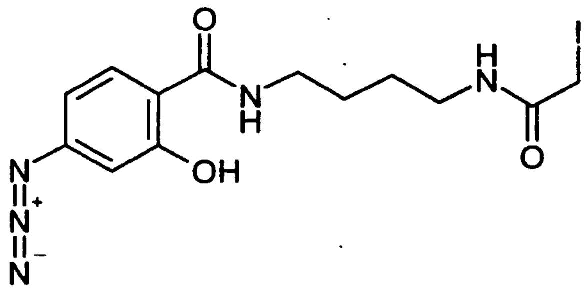

[1-(p-azidosalicylamido)-4-(iodoacetamido)butane], etc.

In view of the above, it is clear that the embodiments where the covalent link is established by means of a reactive side chain or by a heterobifunctional reagent may give rise to a population of complex species where not all species are identical. This is due to the fact that a reactive side chain (e.g. a photo-reactive side chain) and a heterobifunctional reagent (even after specific reaction with an amino acid side chain) may experience more than one potential "reaction partner" upon further reaction, although the number of "reaction partners" may be dramatically reduced if the size (length) of the side chain/reagent is reduced. However in some embodiments it is possible to select the involved amino acid side chains (or bifunctional reagent) in such a manner that virtually on one possible "reaction partner" exist. This is particularly true when the link is in the form of a disulfide bond, because the abundance of possible "reaction partners" (i.e. another cysteine) is very limited.

Hence in a preferred embodiment, the present invention provides the complex as defined hereinabove, wherein the Factor VII polypeptide and the Tissue Factor polypeptide are covalently linked by means of one or more <u>specific links</u> between amino acid side chains of sets of (i) an amino acid of the Factor VII polypeptide and (ii) an amino acid of the Tissue Factor polypeptide.

In the present context, the terms "specific link" and "specific links" are intended to mean that the Factor VII polypeptide and the Tissue Factor polypeptide are covalently linked by means of one or more sets of covalently linked amino acid side chains (possibly via a linker moiety), where the set(s) for at least 90% of the complex species are identical with respect to the position and type of each amino acid within each of the set(s) throughout a population of complex species.

Preferably, at least 95%, or even at least 98%, or even virtually all, of the complex species are identical with respect to the position and type of each amino acid within each of the set(s) throughout a population of complex species.

Even more preferable, the one or more specific links are established as one or more direct bonds between amino acids side chains of one or more sets of an amino acid of the Factor VII polypeptide and an amino acid of the Tissue Factor polypeptide.

Hence the present invention further provides the complex defined hereinabove, wherein the Factor VII polypeptide and the Tissue Factor polypeptide are covalently linked by means of one or more direct bonds between amino acid side chains of sets of (i) an amino acid of the Factor VII polypeptide and (ii) an amino acid of the Tissue Factor polypeptide.

In the present context, the terms "direct bond" and "direct bonds" are intended to mean that the at least one covalent link between an amino acid side chain of the Factor VII polypeptide and an amino acid side chain of the Tissue Factor polypeptide does not include residues derived from cross-linking agents, reagents, etc., but is formed by atoms derived from the respective amino acid side chains, e.g. in the form of an S-S bridge between cysteine amino acid side chains, however, taking into account that such amino acids need not to be natural amino acids, but may, e.g., be photoreactive side chains. Consequently, formation of the direct bond does not include bifunctional reagents and the like.

In view of the above, it is preferred that the sets of amino acids comprise one or more cysteine amino acids.

In one variant, each of the sets comprises one cysteine amino acid, e.g. for use upon coupling to a heterobifunctional reagent.

In another variant, each of the sets comprises two cysteine amino acids. In this variant, disulfide link are established.

It has been found that the proteolytic activity is mainly preserved or even enhanced. Thus, preferred embodiments are those where the proteolytic activity of the Factor VIIa polypeptide is at least 2-fold, such as at least 50-fold, e.g. at least 100-fold, such as at least 200-fold, or at least 250-fold, of that of the free native (wild-type) factor VIIa, as determined by the *in vitro* proteolysis assay defined herein.

Hence, the invention also relates to a covalent complex of a Factor VIIa polypeptide and a Tissue Factor polypeptide, wherein the Factor VIIa polypeptide and the Tissue Factor polypeptide are covalently linked by means of one or more links between amino acid side chains of sets of (i) an amino acid of the Factor VIIa polypeptide and (ii) an amino acid of the Tissue Factor polypeptide, wherein the proteolytic activity of the Factor VIIa polypeptide is at least 2-fold, such as at least 50-fold, e.g. at least 100-fold, such as at least 200-fold, or at least 250-fold, of that of the free native (wild-type) factor VIIa, as determined by the *in vitro* proteolysis assay defined herein.

As mentioned above, the Tissue Factor polypeptide is the soluble ectodomain of Tissue Factor (1-219) or a functional variant or truncated form thereof.

In some important embodiments, at least one of the amino acids in the positions Thr17, Lys20, Ile22, Ser42, Gly43, Asp44, Trp45, Lys46, Ser47, Phe50, Cys57, Asp58, Asp61, Gly109, Gln110, Leu133, Ser205, Pro206 and Val207 in the soluble Tissue Factor polypeptide, in particular Trp45, Asp58, Gly109, Pro206 and Val207 in the soluble Tissue Factor polypeptide, has been replaced by an amino acid with a reactive side chain which gives rise to a designated link, such as a specific link, in particular a direct bond. In a preferred variant, the at least one amino acid is cysteine amino acid.

In other important embodiments, which preferably are combined with the foregoing, at least one of the amino acids in the positions Leu39, Phe40, Ile42, Ser43, Tyr44, Gln64, Leu65, Ile69, Glu77, Gly78, Arg79, Val92, Phe275, Val276, Arg277, Met306, Thr307, and Glu325 of the Factor VII polypeptide, in particular Phe40, Ser43, Gln64 Glu77 and Phe275 of the Factor VII polypeptide, has been replaced by an amino acid with a reactive side chain which gives rise to a designated link, such as a specific link, in particular a direct bond. In a preferred variant, the at least one amino acid is cysteine amino acid.

More particular, one or both of the amino acids of at least one of the amino acid sets

FVII-Ile69 - sTF-Thr17,
FVII-Ile69 - sTF-Lys20,
FVII-Arg79 - sTF-Ile22,
FVII-Val92 - sTF-Ser47,
FVII-Val92 - sTF-Phe50,
FVII-Gly78 - sTF-Cys57,
FVII-Glu77 - sTF-Asp58,
FVII-Gly78 - sTF-Asp58,
FVII-Arg79 - sTF-Asp58,
FVII-Arg277 - sTF-Ser42,
FVII-Val276 - sTF-Gly43,
FVII-Arg277 - sTF-Gly43,
FVII-Phe275 - sTF-Asp44,
FVII-Val276 - sTF-Asp44,
FVII-Arg277 - sTF-Asp44,

FVII-Phe275 - sTF-Trp45,
FVII-Val276 - sTF-Trp45,
FVII-Arg134 - sTF-Trp45,
FVII-Phe275 - sTF-Lys46,
FVII-Gln64 - sTF-Gly109,
FVII-Gln64 - sTF-Gln110,
FVII-Leu65 - sTF-Gln110,
FVII-Phe71 - sTF-Leu133,
FVII-Ser43 - sTF-Ser205,
FVII-Leu39 - sTF-Pro206,
FVII-Phe40 - sTF-Pro206,
FVII-Ile42 - sTF-Pro206,
FVII-Ser43 - sTF-Pro206,
FVII-Tyr44 - sTF-Pro206,
FVII-Leu39 - sTF-Val207,
FVII-Phe40 - sTF-Val207, and
FVII-Ser43 - sTF-Val207,
in particular at least one of the amino acids sets
FVII-Phe40 - sTF-Val207,
FVII-Ser43 - sTF-Pro206,
FVII-Gln64 - sTF-Gly109,
FVII-Glu77 - sTF-Asp58, and
FVII-Phe275 - sTF-Trp45,
have been replaced by an amino acid with a reactive side chain which gives rise to a designated link, such as a specific link, in particular a direct bond.

In a preferred variant, at least one direct bond is a disulfide bond, i.e. a bond between two cysteine amino acids. More preferable, all covalent links are direct bonds in the form of disulfide bonds. In particular, the number of such bonds is 1-5, in particular 1 bond.

In another variant, at least one direct bond is formed via a photo-reactive amino acid, in particular selected from the group consisting of photo-Ile, photo-Leu and photo-Met.

In a still further embodiment, the Factor VII polypeptide and the Tissue Factor polypeptide are covalently linked by means of a linker moiety between amino acid side chains of one or more sets of (i) an amino acid of the Factor VII polypeptide and (i) an amino acid of the Tissue Factor polypeptide. In particular, the linker moiety is derived from a heterobifunctional reagent.

*Preparation of the complex*

*Crosslinking of Tissue factor and factor VII using a heterobifunctional reagent*

In one interesting variant, the method for the preparation of the complex involves production of a cysteine variant of soluble Tissue factor, subsequent labeling of the cysteine in soluble Tissue Factor with a heterobifunctional reagent in which one of the functionalities is cysteine reactive, and finally cross-linking to factor VIIa by virtue of the second functionality of the reagent. Methods for cloning and expression of cysteine variants of Tissue Factor in Escherichia coli as well as subsequent labeling with a cysteine-specific reagent have been described previously (Stone et al. (1995) Biochem. J., 310, 605-614; Freskgård et al. (1996) Protein Sci., 5, 1521-1540; Owenius et al. (1999) Biophys. J., 77, 2237-2250; Österlund et al. (2001) Biochemistry, 40, 9324-9328). Photo-crosslinking of proteins using heterobifunctional reagents containing one cysteine specific and one photo-activatable functionality have been described by Zhang et al. (1995) Biochem. Biophys. Res. Commun., 217, 1177-1184.

*Co-expression of Factor VII polypeptide and Tissue Factor polypeptide*

In one particularly interesting variant, the method for the preparation of the complex involves the coexpression of the Factor VII polypeptide and the Tissue Factor polypeptide, whereby the covalent link between the two polypeptides can be readily established.

More particular, the present invention also relates to a method for production of a complex of a Factor VII polypeptide and a Tissue Factor polypeptide as defined herein, the method comprising a) transfecting a cell with (i) an expression vector comprising a nucleic acid molecule encoding the Factor VII polypeptide and expression control regions operatively linked to thereto and (ii) and an expression vector comprising a nucleic acid molecule encoding the Tissue

Factor polypeptide and expression control regions operatively linked to thereto, b) culturing the transfected cell under conditions for expression of Factor VII polypeptide and Tissue Factor polypeptide, and c) isolating the expressed complex by suitable means.

In one particularly interesting embodiment hereof, the two polypeptides are linked by means of a specific link, more particular by means of a direct link, such as one or more disulfide links between the Factor VII polypeptide and the Tissue Factor polypeptide.

Expression of protein in cells is well-known to the person skilled in the art of protein production. In practicing the method of the invention, the cells are typically eukaryote cells, more preferably an established eukaryote cell line, including, without limitation, CHO (e.g., ATCC CCL 61), COS-1 (e.g., ATCC CRL 1650), baby hamster kidney (BHK), and HEK293 (e.g., ATCC CRL 1573; Graham et al., J. Gen. Virol. 36:59-72, 1977) cell lines. A preferred BHK cell line is the $tk^-$ ts13 BHK cell line (Waechter and Baserga, Proc. Narl. Acad. Sci. USA 79:1106-1110, 1982), hereinafter referred to as BHK 570 cells. The BHK 570 cell line is available from the American Type Culture Collection, 12301 Parklawn Dr., Rockville, MD 20852, under ATCC accession number CRL 10314. A $tk^-$ ts13 BHK cell line is also available from the ATCC under accession number CRL 1632. A preferred CHO cell line is the CHO K1 cell line available from ATCC under accession number CCl61.

Other suitable cell lines include, without limitation, Rat Hep I (Rat hepatoma; ATCC CRL 1600), Rat Hep II (Rat hepatoma; ATCC CRL 1548), TCMK (ATCC CCL 139), Human lung (ATCC HB 8065), NCTC 1469 (ATCC CCL 9.1); DUKX cells (CHO cell line) (Urlaub and Chasin, Proc. Natl. Acad. Sci. USA 77:4216-4220, 1980) (DUKX cells also being referred to as DXB11 cells), and DG44 (CHO cell line) (Cell, 33: 405, 1983, and Somatic Cell and Molecular Genetics 12: 555, 1986). Also useful are 3T3 cells, Namalwa cells, myelomas and fusions of myelomas with other cells. In some embodiments, the cells may be mutant or recombinant cells, such as, e.g., cells that express a qualitatively or quantitatively different spectrum of enzymes that catalyze post-translational modification of proteins (e.g., glycosylation enzymes such as glycosyl transferases and/or glycosidases, or processing enzymes such as propeptides) than the cell type from which they were derived. Suitable insect cell lines also include, without limitation, Lepidoptera cell lines, such as Spodoptera frugiperda cells or Trichoplusia ni cells (see, e.g., US 5,077,214).

In some embodiments, the cells used in practicing the invention are capable of growing in suspension cultures. As used herein, suspension-competent cells are those that can grow in suspension without making large, firm aggregates, i.e., cells that are monodisperse or grow in loose aggregates with only a few cells per aggregate. Suspension-competent cells include, without limitation, cells that grow in suspension without adaptation or manipulation (such as, e.g., hematopoietic cells or lymphoid cells) and cells that have been made suspension-competent by gradual adaptation of attachment-dependent cells (such as, e.g., epithelial or fibroblast cells) to suspension growth.

The cells used in practicing the invention may be adhesion cells (also known as anchorage-dependent or attachment-dependent cells). As used herein, adhesion cells are those that need to adhere or anchor themselves to a suitable surface for propagation and growth. In one embodiment of the invention, the cells used are adhesion cells. In these embodiments, both the propagation phases and the production phase include the use of microcarriers. The used adhesion cells should be able to migrate onto the carriers (and into the interior structure of the carriers if a macroporous carrier is used) during the propagation phase(s) and to migrate to new carriers when being transferred to the production bioreactor. If the adhesion cells are not sufficiently able to migrate to new carriers by themselves, they may be liberated from the carriers by contacting the cell-containing microcarriers with proteolytic enzymes or EDTA. The medium used (particularly when free of animal-derived components) should furthermore contain components suitable for supporting adhesion cells; suitable media for cultivation of adhesion cells are available from commercial suppliers, such as, e.g., Sigma.

The cells may also be suspension-adapted or suspension-competent cells. If such cells are used, the propagation of cells may be done in suspension, thus microcarriers are only used in the final propagation phase in the production culture vessel itself and in the production phase. In case of suspension-adapted cells the microcarriers used are typically macroporous carriers wherein the cells are attached by means of physical entrapment inside the internal structure of the carriers. In such embodiments, the eukaryotic cell is typically selected from CHO, BHK, HEK293, myeloma cells, etc.

The Factor VII polypeptide and the Tissue Factor polypeptide may also be co-expressed in E. coli. or in bacteria or in transgenic animals, e.g. as discloses in the international patent application with publication number WO 05/075635.

## EXAMPLES

The terminology for amino acid substitutions used in the following examples is as follows. The first letter represents the amino acid naturally present at a position of SEQ ID NO:1 or SEQ ID NO:2. The following number represents the position in SEQ ID NO:1 or SEQ ID NO:2. The second letter represents the different amino acid substituting for the natural amino acid. An example is factor VIIa F40C, where a phenylalanine at position 40 of SEQ ID No:1 is replaced by a cysteine. In another example, sTF(1-219) V207C, the valine in position 207 of SEQ ID NO:2 is replaced by a cysteine.

**Example 1**

*Materials* - D-Phe-Phe-Arg-chloromethyl ketone was purchased from Bachem. Chromogenic Z-D-Arg-Gly-Arg-p-nitroanilide (S-2765), and H-D-Ile-Pro-Arg-p-nitroanilide (S-2288) substrates were obtained from Chromogenix (Sweden). Human plasma-derived factor X (hFX), Factor Xa (hFXa), and factor IXa (hFIXa) were obtained from Enzyme Research Laboratories Ltd. (South Bend, IN). Human whole brain Marathon-ready cDNA library was obtained from Clontech (Mountain View, CA). Soluble tissue factor 1-219 (sTF(1-219)) expressed in *Escherichia coli* was prepared according to published procedures (Freskgård et al. (1996) Protein Sci., 5, 1531-1540). Expression and purification of recombinant factor VIIa was performed as described previously (Thim et al. (1988) Biochemistry, 27, 7785-7793; Persson et al. (1996) FEBS Lett., 385, 241-243). All other chemicals were of analytical grade or better.

*Construction of DNA encoding factor VII and factor VII F40C mutant* - The DNA coding sequence of factor VII including its pre (signal sequence) and pro-regions was amplified by PCR using Expand High Fidelity PCR system (Roche Diagnostics Corporation, Indianapolis, IN) according to manufacturer's recommendations and primers oHOJ104-f and oHOJ104-r, introducing flanking *Nhe*I and *Not*I restriction sites (primer sequences are listed in Table 1). The DNA template for the PCR reaction was pLN174 as disclosed in international patent application with publication number WO 02/077218. The purified PCR product was cut with NheI and NotI and then ligated into the corresponding sites of pCI-neo (Promega, Madison, WI) to give pHOJ300. The amino acid of native (wild-type) factor VII is given in SEQ ID NO:1. The DNA sequence of native (wild-type) factor VII including its pre (signal sequence) and pro-regions is given in SEQ ID NO:3.

Plasmid pHOJ344 encoding factor VII F40C was constructed by QuickChange® Site-Directed Mutagenesis using primers oHOJ143-f and oHOJ143-r and pHOJ300 as template according to manufacturer's instructions (Stratagene, La Jolla, CA). The correct identity of all cloned sequences was verified by DNA sequencing.

*Construction of DNA encoding sTF(1-219) and sTF(1-219) V207C mutant* - The DNA coding sequence of sTF (1-219) including its signal sequence was amplified from a human whole brain cDNA library (Marathon-ready cDNA; Clontech Laboratories Inc., Mountain View, CA) by PCR using Expand High Fidelity PCR system (Roche Diagnostics Corporation, Indianapolis, IN) according to manufacturer's recommendations and primers oHOJ152-f and oHOJ152-r, introducing flanking *Nhe*I and *Xho*I restriction sites (primer sequences are listed in Table 1). The purified PCR product was cut with NheI and XhoI and then ligated into the corresponding sites of pCI-neo (Promega, Madison, WI) to give pHOJ356.

**Table 1** - DNA oligos used for construction of plasmids.

| Primer | Plasmid | Sequence (5'→3') |
|---|---|---|
| oHOJ104-f | pHOJ300 | GGCGGCGGCTAGCATGGTCTCCCAGGCCCTCAGGCTCCTCTGCC |
| oHOJ104-r | pHOJ300 | CCGCCGCCGCGGCCGCCTAGGGAAATGGGGCTCGCAGGAGG |
| oHOJ143-f | pHOJ344 | GCGGAGAGGACGAAGCTGTGCTGGATTTCTTACAGTGATG |
| oHOJ143-r | pHOJ344 | CATCACTGTAAGAAATCCAGCACAGCTTCGTCCTCTCCGC |
| oHOJ152-f | pHOJ356 | GGCGGCGGGCTAGCATGGAGACCCCTGCCTGGCCCCGG |
| oHOJ152-r | pHOJ356 | CCGCCGCCCTCGAGTTATTCTCTGAATTCCCCTTTCTCCTGG |
| oHOJ144-f | pHOJ357 | GAGTACAGACAGCCCGTGCGAGTGTATGGGCCAG |
| oHOJ144-r | pHOJ357 | CTGGCCCATACACTCGCACGGGCTGTCTGTACTC |

The amino acid of sTF(1-219) is given in SEQ ID NO:4. The DNA sequence of sTF(1-219) including its signal sequence is given in SEQ ID NO:5.

Plasmid pHOJ357 encoding sTF(1-219) V207C was constructed by QuickChange® Site-Directed Mutagenesis using primers oHOJ144-f and oHOJ144-r and pHOJ356 as template according to manufacturer's instructions (Stratagene, La Jolla, CA). The correct identity of all cloned sequences was verified by DNA sequencing.

*Co-expression of factor VII F40C and sTF(1-219) V207C* - Factor VIIa F40C and sTF(1-219) V207C were co-expressed by transient expression in the Freestyle™ 293 Expression System (Invitrogen, Carlsbad, CA) as instructed by the manufacturer. Briefly, HEK293F cells (Invitrogen, Carlsbad, CA) were propagated from a cryo-preserved culture by first establishing a 72-ml shaker culture of $1.5 \times 10^7$ cells in FreeStyle™ 293 Expression Medium (Invitrogen, Carlsbad, CA). The culture was then expanded to 2000 ml of $2.2 \times 10^9$ cells over a period of 12 days. Cells were grown in baffled conical PEGT flasks with loose caps (Nunc, Denmark) at 37°C, 8% CO2, and 100-125 rpm in an orbital shaker incubator (Multitron II, Infors, Switzerland). The cell density never exceeded $1.4 \times 10^6$ cells/ml before expansion of the culture, and

the flask:culture volume was kept at approximately 3: 1. Except for the initial 72-ml culture, the provided growth medium was supplemented with vitamin K1 to 5 ppm (Sigma) required for post-translational gamma-carboxylation of factor VII. The expanded HEK293F culture (2000 ml, $2.2x10^9$ cells) was then transfected with a mixture containing 800 μg of plasmid pHOJ344 (1μg/μl) and 1500 μg of plasmid DNA pHOJ357 (1μg/μl). Addition of plasmid DNA and 239fectin™ (Invitrogen, Carlsbad, CA) to the Opti-MEM® I solution (Invitrogen, Carlsbad, CA), as well as mixing of these and the subsequent addition of the formed lipid DNA complexes to the cell culture were performed dropwise while gently swirling the receiving solution and according to manufacturer's instructions. Following incubation of the transfected culture for 96 hours at 37°C, 8% CO2, and 100 rpm, the conditioned growth medium containing the factor VIIa F40C-sTF(1-219) V207C complex was recovered by centrifugation and stored at -80°C until further processing.

*Purification of Factor VII F40C-sTF(1-219) V207C complex* - Conditioned medium to which $CaCl_2$ had been added to a concentration of 10mM was loaded onto a 25-ml column containing the monoclonal antibody F1A2 (Novo Nordisk A/S, Bagsvaerd, Denmark) coupled to CNBr-activated Sepharose 4B (Amersham Biosciences, GE Healthcare). The column was equilibrated with 50mM HEPES, 100mM NaCl, 10mM $CaCl_2$, pH 7.5. After washing with equilibration buffer and equilibration buffer containing 2 M NaCl, bound material was eluted with equilibration buffer containing 10mM EDTA instead of $CaCl_2$. Calcium chloride was subsequently added to the collected peak fraction to a final concentration of 20mM.

To remove small amounts of free factor VIIa F40C, the preparation was passed over a 1-ml HiTrap NHS column (GE Healthcare) to which 4 mg sTF(1-219) had been coupled according to manufacturer's instructions. Prior to loading, the column was equilibrated in 50mM HEPES, 100mM NaCl, 10mM $CaCl_2$, pH 7.5. The flow through containing factor VII F40C-sTF(1-219) V207C complex, and devoid of detectable free factor VII F40C and sTF(1-219) V207C, was collected.

To promote activation of the factor VII F40C-sTF(1-219) V207C complex, human factor IXa was added to a final concentration of 0.1 mg/ml. After complete activation as verified by reducing SDS-PAGE, factor VIIa F40C-sTF (1-219) V207C complex was purified by F1A2 Sepharose 4B affinity chromatography as described above, except that a 5-ml column was used and the equilibration buffer was 10mM MES, 100mM NaCl, 10mM $CaCl_2$, pH 6.0. The final protein preparation was stored in aliquots at -80°C.

*Active-site titration assay* - Active site concentrations of native (wild-type) factor VIIa and factor VIIa F40C-sTF (1-219) V207C were determined from the irreversible loss of amidolytic activity upon titration with sub-stoichiometric levels of of D-Phe-Phe-Arg-chloromethyl ketone (FFR-cmk) essentially as described elsewhere (Bock P.E. (1992) J.Biol.Chem., 267, 14963-14973). Briefly, each protein was diluted into 50 mM HEPES, 100 mM NaCl, 10 mM $CaCl_2$, 0.01% Tween 80, pH 7.0 buffer to an approximate concentration of 300 nM. Diluted protein (20 μl) was then combined with 20 μl 1.5 μM sTF in the case of free factor VIIa and 20 μl buffer in the case of factor VIIa F40C-sTF(1-219) V207C, and finally 20 μl 0-1.2 μM FFR-cmk (freshly prepared in buffer from a FFR-cmk stock dissolved in DMSO and stored at -80°C). After overnight incubation at room temperature, residual amidolytic activity was measured. The activity assay was carried out in polystyrene microtiter plates (Nunc, Denmark) in a final volume of 200 μl assay buffer (50 mM HEPES, 100 mM NaCl, 5 mM $CaCl_2$, 0.01% Tween 80, pH 7.4) containing approx. 10 nM factor VIIa or factor VIIa-sTF complex, corresponding to 10-fold dilutions of the samples. After 15 min pre-incubation at room temperature, 1 mM chromogenic substrate S-2288 was added and the absorbance monitored continuously at 405 nm for 10 min in a SpectraMax™ 340 microplate spectrophotometer equipped with SOFTmax PRO software (v2.2; Molecular Devices Corp., Sunnyvale, CA). Amidolytic activity was reported as the slope of the linear progress curves after blank subtraction. Active site concentrations were determined by extrapolation to zero activity, corresponding to the minimal concentration of FFR-cmk completely abolishing amidolytic activity.

*In vitro proteolysis assay* - Native (wild-type) factor VIIa and factor VIIa F40C-sTF(1-219) V207C were assayed in parallel to directly compare their specific activities. The assay was carried out in a microtiter plate (Nunc, Denmark). Factor VIIa (300 nM) or factor VIIa F40C-sTF(1-219) V207C (15 nM) and human Factor X (0.2 μM) in 100 μl 50mM HEPES, 100mM NaCl, 5mM $CaCl_2$, 0.01% Tween 80, pH 7.4 buffer were incubated for 20 min at ambient temperature. Factor X activation was then stopped by the addition of 50 μl 50 mM HEPES, 100 mM NaCl, 40 mM EDTA, 0.01% Tween 80, pH 7.4 buffer containing 0.5 μM TF8-5G9 antibody blocking the FX binding site on TF (Ruf et al. (1991) Thromb. Haemost., 66, 529-533). The amount of FXa generated was measured by addition of 50 μl of the chromogenic substrate Z-D-Arg-Gly-Arg-p-nitroanilide (S-2765) to a final concentration 0.5 mM. The absorbance at 405 nm was measured continuously in a SpectraMax™ 340 microplate spectrophotometer equipped with SOFTmax PRO software (v2.2; Molecular Devices Corp., Sunnyvale, CA). Specific amidolytic activities, reported as the slope of the linear progress curves after blank subtraction divided by the protein concentration in the assay, were used to calculate the ratio between the specific proteolytic activities of factor VIIa-sTF complex and wild-type factor VIIa:

**Ratio = (($A_{405\,nm}$ factor VIIa-sTF complex)/[Factor VIIa-sTF complex]) / (( $A_{405\,nm}$ factor VIIa wild-type)/[Factor VIIa wild-type])**

Results are given in Table 2.

**Table 2** - Relative proteolytic activities as described in the *in vitro* proteolysis assay

| Protein | Relative proteolytic activity (ratio) |
|---|---|
| Factor VIIa F40C-STF(1-219) V207C | 65.5 |
| Wild-type factor VIIa | 1 |

*SDS-PAGE analysis* - Factor VIIa and factor VIIa F40C-sTF(1-219) V207C (approx 1.5 μg of each) were analyzed by non-reducing and reducing SDS-PAGE on a 4-12% Bis-Tris NuPAGE® gel (Invitrogen Life Technologies, Carlsbad, CA) run at 200 V for 35 min in MES buffer (Invitrogen Life Technologies, Carlsbad, CA) according to manufacturer's recommendations. Gels were washed with water and stained with Simply Blue™ SafeStain (Invitrogen Life Technologies, Carlsbad, CA) according to manufacturer's recommendations. The gel is shown in Figure 2.

*SEQ ID NO:1* - Amino acid sequence (1-406) of native (wild-type) human factor VII. The three-letter indication "GLA" means 4-carboxyglutamic acid (γ-carboxyglutamate)

Ala$^{1}$-Asn-Ala-Phe-Leu-GLA-GLA-Leu-Arg-Pro$^{10}$-Gly-Ser-Leu-GLA-Arg-GLA-Cys-Lys-GLA-GLA$^{20}$-Gln-Cys-Ser-Phe-GLA-GLA-Ala-Arg-GLA-Ile$^{30}$-Phe-Lys-Asp-Ala-GLA-Arg-Thr-Lys-Leu-Phe$^{40}$-Trp-Ile-Ser-Tyr-Ser-Asp-Gly-Asp-Gln-Cys$^{50}$-Ala-Ser-Ser-Pro-Cys-Gln-Asn-Gly-Gly-Ser$^{60}$-Cys-Lys-Asp-Gln-Leu-Gln-Ser-Tyr-Ile-Cys$^{70}$-Phe-Cys-Leu-Pro-Ala-Phe-Glu-Gly-Arg-Asn$^{80}$-Cys-Glu-Thr-His-Lys-Asp-Asp-Gln-Leu-Ile$^{90}$-Cys-Val-Asn-Glu-Asn-Gly-Gly-Cys-Glu-Gln$^{100}$-Tyr-Cys-Ser-Asp-His-Thr-Gly-Thr-Lys-Arg$^{110}$-Ser-Cys-Arg-Cys-His-Glu-Gly-Tyr-Ser-Leu$^{120}$-Leu-Ala-Asp-Gly-Val-Ser-Cys-Thr-Pro-Thr$^{130}$-Val-Glu-Tyr-Pro-Cys-Gly-Lys-Ile-Pro-Ile$^{140}$-Leu-Glu-Lys-Arg-Asn-Ala-Ser-Lys-Pro-Gln$^{150}$-Gly-Arg-Ile-Val-Gly-Gly-Lys-Val-Cys-Pro$^{160}$-Lys-Gly-Glu-Cys-Pro-Trp-Gln-Val-Leu-Leu$^{170}$-Leu-Val-Asn-Gly-Ala-Gln-Leu-Cys-Gly-Gly$^{180}$-Thr-Leu-Ile-Asn-Thr-Ile-Trp-Val-Val-Ser$^{190}$-Ala-Ala-His-Cys-Phe-Asp-Lys-Ile-Lys-Asn$^{200}$-Trp-Arg-Asn-Leu-Ile-Ala-Val-

Leu-Gly-Glu$^{210}$-His-Asp-Leu-Ser-Glu-His-Asp-Gly-Asp-Glu$^{220}$-Gln-Ser-Arg-Arg-Val-Ala-Gln-Val-Ile-Ile$^{230}$-Pro-Ser-Thr-Tyr-Val-Pro-Gly-Thr-Thr-Asn$^{240}$-His-Asp-Ile-Ala-Leu-Leu-Arg-Leu-His-Gln$^{250}$-Pro-Val-Val-Leu-Thr-Asp-His-Val-Val-Pro$^{260}$-Leu-Cys-Leu-Pro-Glu-Arg-Thr-Phe-Ser-Glu$^{270}$-Arg-Thr-Leu-Ala-Phe-Val-Arg-Phe-Ser-Leu$^{280}$-Val-Ser-Gly-Trp-Gly-Gln-Leu-Leu-Asp-Arg$^{290}$-Gly-Ala-Thr-Ala-Leu-Glu-Leu-Met-Val-Leu$^{300}$-Asn-Val-Pro-Arg-Leu-Met-Thr-Gln-Asp-Cys$^{310}$-Leu-Gln-Gln-Ser-Arg-Lys-Val-Gly-Asp-Ser$^{320}$-Pro-Asn-Ile-Thr-Glu-Tyr-Met-Phe-Cys-Ala$^{330}$-Gly-Tyr-Ser-Asp-Gly-Ser-Lys-Asp-Ser-Cys$^{340}$-Lys-Gly-Asp-Ser-Gly-Gly-Pro-His-Ala-Thr$^{350}$-His-Tyr-Arg-Gly-Thr-Trp-Tyr-Leu-Thr-Gly$^{360}$-Ile-Val-Ser-Trp-Gly-Gln-Gly-Cys-Ala-Thr$^{370}$-Val-Gly-His-Phe-Gly-Val-Tyr-Thr-Arg-Val$^{380}$-Ser-Gln-Tyr-Ile-Glu-Trp-Leu-Gln-Lys-Leu$^{390}$-Met-Arg-Ser-Glu-Pro-Arg-Pro-Gly-Val-Leu$^{400}$-Leu-Arg-Ala-Pro-Phe-Pro$^{406}$

*SEQ ID NO:3* - DNA sequence of native (wild-type) factor VII including its pre (signal sequence) and pro-regions (underlined).

ATGGTCTCCCAGGCCCTCAGGCTCCTCTGCCTTCTGCTTGGGCTTCAGGCTGCCTGGCTGCAGTCTT
CGTAACCCAGGAGGAAGCCCAAGGCGTCCTGCACCGGCGCCGGCGCGCCAACGCGTTCCTGGAGGA
GCTGCGGCCGGGCTCCCTGGAGAGGGAGTGCAAGGAGGAGCAGTGCTCCTTCGAGGAGGCCCGGG
AGATCTTCAAGGACGCGGAGAGGACGAAGCTGTTCTGGATTTCTTACAGTGATGGGGACCAGTGTGC
CTCAAGTCCATGCCAGAATGGGGGCTCCTGCAAGGACCAGCTCCAGTCCTATATCTGCTTCTGCCTCC
CTGCCTTCGAGGGCCGGAACTGTGAGACGCACAAGGATGACCAGCTGATCTGTGTGAACGAGAACGG
CGGCTGTGAGCAGTACTGCAGTGACCACACGGGCACCAAGCGCTCCTGTCGGTGCCACGAGGGGTA
CTCTCTGCTGGCAGACGGGGTGTCCTGCACACCCACAGTTGAATATCCATGTGGAAAAATACCTATTC
TAGAAAAAAGAAATGCCAGCAAACCCCAAGGCCGAATTGTGGGGGGCAAGGTGTGCCCCAAAGGGG
AGTGTCCATGGCAGGTCCTGTTGTTGGTGAATGGAGCTCAGTTGTGTGGGGGGACCCTGATCAACAC
CATCTGGGTGGTCTCCGCGGCCCACTGTTTCGACAAAATCAAGAACTGGAGGAACCTGATCGCGGTG
CTGGGCGAGCACGACCTCAGCGAGCACGACGGGGATGAGCAGAGCCGGCGGGTGGCGCAGGTCAT
CATCCCCAGCACGTACGTCCCGGGCACCACCAACCACGACATCGCGCTGCTCCGCCTGCACCAGCCC
GTGGTCCTCACTGACCATGTGGTGCCCCTCTGCCTGCCCGAACGGACGTTCTCTGAGAGGACGCTGG
CCTTCGTGCGCTTCTCATTGGTCAGCGGCTGGGGCCAGCTGCTGGACCGTGGCGCCACGGCCCTGGA
GCTCATGGTCCTCAACGTGCCCCGGCTGATGACCCAGGACTGCCTGCAGCAGTCACGGAAGGTGGGA
GACTCCCCAAATATCACGGAGTACATGTTCTGTGCCGGCTACTCGGATGGCAGCAAGGACTCCTGCAA
GGGGGACAGTGGAGGCCCACATGCCACCCACTACCGGGGCACGTGGTACCTGACGGGCATCGTCAG
CTGGGGCCAGGGCTGCGCAACCGTGGGCCACTTTGGGGTGTACACCAGGGTCTCCCAGTACATCGA
GTGGCTGCAAAAGCTCATGCGCTCAGAGCCACGCCCAGGAGTCCTCCTGCGAGCCCCATTTCCCTAG

*SEQ ID NO:4* - Amino acid sequence of sTF(1-219)

Ser$^{1}$-Gly-Thr-Thr-Asn-Thr-Val-Ala-Ala-Tyr$^{10}$-Asn-Leu-Thr-Trp-Lys-Ser-Thr-Asn-Phe-Lys$^{20}$-Thr-Ile-Leu-Glu-Trp-Glu-Pro-Lys-Pro-Val$^{30}$-Asn-Gln-Val-Tyr-Thr-Val-Gln-Ile-Ser-Thr$^{40}$-Lys-Ser-Gly-Asp-Trp-Lys-Ser-Lys-Cys-Phe$^{50}$-Tyr-Thr-Thr-Asp-Thr-Glu-Cys-Asp-Leu-Thr$^{60}$-Asp-Glu-Ile-

Val-Lys-Asp-Val-Lys-Gln-Thr$^{70}$-Tyr-Leu-Ala-Arg-Val-Phe-Ser-Tyr-Pro-Ala$^{80}$-Gly-Asn-Val-Glu-Ser-Thr-Gly-Ser-Ala-Gly$^{90}$-Glu-Pro-Leu-Tyr-Glu-Asn-Ser-Pro-Glu-Phe$^{100}$-Thr-Pro-Tyr-Leu-Glu-Thr-Asn-Leu-Gly-Gln$^{110}$-Pro-Thr-Ile-Gln-Ser-Phe-Glu-Gln-Val-Gly$^{120}$-Thr-Lys-Val-Asn-Val-Thr-Val-Glu-Asp-Glu$^{130}$-Arg-Thr-Leu-Val-Arg-Arg-Asn-Asn-Thr-Phe$^{140}$-Leu-Ser-Leu-Arg-Asp-Val-Phe-Gly-Lys-Asp$^{150}$-Leu-Ile-Tyr-Thr-Leu-Tyr-Tyr-Trp-Lys-Ser$^{160}$-Ser-Ser-Ser-Gly-Lys-Lys-Thr-Ala-Lys-Thr$^{170}$-Asn-Thr-Asn-Glu-Phe-Leu-Ile-Asp-Val-Asp$^{180}$-Lys-Gly-Glu-Asn-Tyr-Cys-Phe-Ser-Val-Gln$^{190}$-Ala-Val-Ile-Pro-Ser-Arg-Thr-Val-Asn-Arg$^{200}$-Lys-Ser-Thr-Asp-Ser-Pro-Val-Glu-Cys-Met$^{210}$-Gly-Gln-Glu-Lys-Gly-Glu-Phe-Arg-Glu$^{219}$

*SEQ ID NO:5* - DNA sequence of sTF(1-219) including its signal sequence (underlined)

ATGGAGACCCCTGCCTGGCCCCGGGTCCCGCGCCCCGAGACCGCCGTCGCTCGGACGCTCCTGCTC
GGCTGGGTCTTCGCCCAGGTGGCCGGCGCTTCAGGCACTACAAATACTGTGGCAGCATATAATTTAAC
TTGGAAATCAACTAATTTCAAGACAATTTTGGAGTGGGAACCCAAACCCGTCAATCAAGTCTACACTGT
TCAAATAAGCACTAAGTCAGGAGATTGGAAAAGCAAATGCTTTTACACAACAGACACAGAGTGTGACC
TCACCGACGAGATTGTGAAGGATGTGAAGCAGACGTACTTGGCACGGGTCTTCTCCTACCCGGCAGG
GAATGTGGAGAGCACCGGTTCTGCTGGGGAGCCTCTGTATGAGAACTCCCCAGAGTTCACACCTTAC
CTGGAGACAAACCTCGGACAGCCAACAATTCAGAGTTTTGAACAGGTGGGAACAAAAGTGAATGTGA
CCGTAGAAGATGAACGGACTTTAGTCAGAAGGAACAACACTTTCCTAAGCCTCCGGGATGTTTTTGGC
AAGGACTTAATTTATACACTTTATTATTGGAAATCTTCAAGTTCAGGAAAGAAAACAGCCAAAACAAAC
ACTAATGAGTTTTTGATTGATGTGGATAAAGGAGAAAACTACTGTTTCAGTGTTCAAGCAGTGATTCCC
TCCCGAACAGTTAACCGGAAGAGTACAGACAGCCCGGTAGAGTGTATGGGCCAGGAGAAAGGGGAA
TTCAGAGAATAA

**Example 2**

*Construction of DNA encoding sTF(1-219) W45C, sTF(1-219) D58C, sTF(1-219) G109C, and sTF(1-219) P206C* - Mutants of sTF(1-219) are constructed by QuickChange® Site-Directed Mutagenesis using primer pairs (forward and reverse) listed in Table 3 and pHOJ356 as template according to manufacturer's instructions (Stratagene, La Jolla, CA). The correct identity of all cloned sequences is verified by DNA sequencing.

**Table 3** - Forward and reverse primers (oligos) used for site-directed mutagenesis of TF cDNA.

| Primer | Mutation | Sequence (5'→3') |
|---|---|---|
| oHOJ172-f | W45C | CACTAAGTCAGGAGATTGCAAAAGCAAATGCTTTTAC |
| oHOJ172-r | W45C | GTAAAAGCATTTGCTTTTGCAATCTCCTGACTTAGTG |
| oHOJ173-f | D58C | CAACAGACACAGAGTGTTGCCTCACCGACGAGATTG |
| oHOJ173-r | D58C | CAATCTCGTCGGTGAGGCAACACTCTGTGTCTGTTG |
| oHOJ174-f | G109C | CCTGGAGACAAACCTCTGCCAGCCAACAATTCAGAG |
| oHOJ174-r | G109C | CTCTGAATTGTTGGCTGGCAGAGGTTTGTCTCCAGG |
| oHOJ175-f | P206C | GAAGAGTACAGACAGCTGCGTAGAGTGTATGGGC |
| oHOJ175-r | P206C | GCCCATACACTCTACGCAGCTGTCTGTACTCTTC |

*Construction of DNA encoding factor VII S43C, factor VII Q64C, factor VII E77C, and factor VII F275C* - Mutants of factor VII are constructed by QuickChange® Site-Directed Mutagenesis using primer pairs (forward and reverse) listed in Table 4 and pHOJ300 as template according to manufacturer's instructions (Stratagene, La Jolla, CA). The correct identity of all cloned sequences is verified by DNA sequencing.

**Table 4 -** Forward and reverse primers (oligos) used for site-directed mutagenesis of factor VII cDNA.

| Primer | Mutation | Sequence (5'→3') |
|---|---|---|
| oHOJ23-f | S43C | GGACGAAGCTGTTCTGGATTTGCTACAGTGATGGGGAC |
| oHOJ23-r | S43C | GTCCCCATCACTGTAGCAAATCCAGAACAGCTTCGTCC |
| oHOJ20-f | Q64C | GGGCTCCTGCAAGGACTGCCTCCAGTCCTATATCTG |
| oHOJ20-r | Q64C | CAGATATAGGACTGGAGGCAGTCCTTGCAGGAGCCC |
| oHOJ176-f | E77C | CTGCCTCCCTGCCTTCTGCGGCCGGAACTGTGAG |
| oHOJ176-r | E77C | CTCACAGTTCCGGCCGCAGAAGGCAGGGAGGCAG |
| oHOJ177-f | F275C | GAGAGGACGCTGGCCTGCGTGCGCTTCTCATTG |
| oHOJ177-r | F275C | CAATGAGAAGCGCACGCAGGCCAGCGTCCTCTC |

*Co-expression of factor VII S43C-sTF(1-219) P206C, factor VII Q64C-sTF(1-219) G109C, factor VII E77C-sTF(1-219) D58C, and factor VII F275C-sTF(1-219) W45C* - Cysteine variants of factor VIIa and sTF(1-219) are co-expressed by transient expression in the Freestyle™ 293 Expression System (Invitrogen, Carlsbad, CA) as instructed by the manufacturer and detailed in Example 1.

*Immuno-blot detection of factor VII Q64C-sTF(1-219) G109C* - Conditioned medium from transient co-expression of factor VII Q64C and sTF(1-219) G109C was analyzed by non-reducing SDS-PAGE on a 4-12% Bis-Tris NuPAGE® gel (Invitrogen Life Technologies, Carlsbad, CA) run at 200 V for 35 min in MES buffer (Invitrogen Life Technologies, Carlsbad, CA) according to manufacturer's recommendations. Complexes were subsequently detected by immunoblotting as known to people skilled in the art using a mixture of monoclonal mouse anti-human FVIIa and anti-human TF primary antibodies and secondary horse radish peroxidase (HRP) conjugated rabbit anti-mouse antibody. Immune-complexes were detected using the SuperSignal® West Pico Chemiluminescent detection kit (Pierce) and a LAS-3000 Image Reader (FUJIFILM Corporation). Results are shown in Figure 3.

**Example 3**

*Construction of DNA encoding sTF(1-209) and sTF(1-209) V207C* - The DNA coding sequence of TF(1-209) including its signal peptide is amplified from a human whole brain cDNA library (Marathon-ready cDNA; Clontech Laboratories Inc., Mountain View, CA) by PCR using Expand High Fidelity PCR system (Roche Diagnostics Corporation, Indianapolis, IN) according to manufacturer's recommendations and primers oHOJ152-f (sequence listed in Table 1) and oHOJ178-r (sequence listed in Table 5), introducing flanking *Nhe*I and *Xho*I restriction sites. The purified PCR product is cut with *Nhe*I and *Xho*I and then ligated into the corresponding sites of pCI-neo (Promega, Madison, WI). The resulting plasmid then serves as template for the construction of sTF(1-209) V207C using QuickChange® Site-Directed

Mutagenesis and primers oHOJ179-f and oHOJ179-r (sequences listed in Table 5) according to manufacturer's instructions (Stratagene, La Jolla, CA). The correct identity of all cloned sequences is verified by DNA sequencing.

**Table 5** - Primers for construction of TF(1-209) and TF(1-209) V207C DNA

| **Primer** | **Sequence (5'→3')** |
|---|---|
| oHOJ177-r | CCGCCGCCCTCGAGTTAACACTCTACCGGGCTGTCTGTACTC |
| oHOJ179-f | GAGTACAGACAGCCCGTGCGAGTGTTAACTCGAG |
| oHOJ179-r | CTCGAGTTAACACTCGCACGGGCTGTCTGTACTC |

**Claims**

**1.** A covalent complex of a functionally active Factor VII polypeptide and a Tissue Factor polypeptide, wherein said Factor VII polypeptide and Tissue Factor polypeptide are covalently linked by means of one or more direct bonds between amino acid side chains of sets of (i) an amino acid of the Factor VII polypeptide and (ii) an amino acid of the Tissue Factor polypeptide.

**2.** The complex according to claim 1, wherein the proteolytic activity of the Factor VII polypeptide is at least 2-fold, such as at least 50-fold, e.g. at least 100-fold, such as at least 200-fold, or at least 250-fold, of that of the free native (wild-type) factor VIIa, as determined by the *in vitro* proteolysis assay.

**3.** The complex according to any one of the preceding claims, wherein the sets of amino acids comprise one or more cysteine amino acids.

**4.** The complex according to claim 3, wherein each of the sets comprises one cysteine amino acid.

**5.** The complex according to claim 3, wherein each of the sets comprises two cysteine amino acids.

**6.** The complex according to any one of the preceding claims, wherein at least one of the amino acids in positions Thr17, Lys20, Ile22, Ser42, Gly43, Asp44, Trp45, Lys46, Ser47, Phe50, Cys57, Asp58, Asp61, Gly109, Gln110, Leu133, Ser205, Pro206 and Val207 of the soluble Tissue Factor polypeptide, in particular Trp45, Asp58, Gly109, Pro206 and Val207 of the soluble Tissue Factor polypeptide, has been replaced by an amino acid with a reactive side chain which gives rise to said direct bond(s).

**7.** The complex according to any one of the preceding claims, wherein at least one of the amino acids in positions Leu39, Phe40, Ile42, Ser43, Tyr44, Gln64, Leu65, Ile69, Glu77, Gly78, Arg79, Val92, Phe275, Val276, Arg277, Met306, Thr307, and Glu325 of the Factor VII polypeptide, in particular Phe40, Ser43, Gln64 Glu77 and Phe275 of the Factor VII polypeptide, has been replaced by an amino acid with a reactive side chain which gives rise to said direct bond(s).

**8.** The complex according to any one of the preceding claims, wherein one or both of the amino acids of at least one of the amino acid sets
FVII-Ile69 - sTF-Thr17,
FVII-Ile69 - sTF-Lys20,
FVII-Arg79 - sTF-Ile22,
FVII-Val92 - sTF-Ser47,
FVII-Val92 - sTF-Phe50,
FVII-Gly78 - sTF-Cys57,
FVII-Glu77 - sTF-Asp58,
FVII-Gly78 - sTF-Asp58,
FVII-Arg79 - sTF-Asp58,
FVII-Arg277 - sTF-Ser42,
FVII-Val276 - sTF-Gly43,

FVII-Arg277 - sTF-Gly43,
FVII-Phe275 - sTF-Asp44,
FVII-Val276 - sTF-Asp44,
FVII-Arg277 - sTF-Asp44,
FVII-Phe275 - sTF-Trp45,
FVII-Val276 - sTF-Trp45,
FVII-Argl34 - sTF-Trp45,
FVII-Phe275 - sTF-Lys46,
FVII-Gln64 - sTF-Gly109,
FVII-Gln64 - sTF-Gln110,
FVII-Leu65 - sTF-Gln110,
FVII-Phe71 - sTF-Leu133,
FVII-Ser43 - sTF-Ser205,
FVII-Leu39 - sTF-Pro206,
FVII-Phe40 - sTF-Pro206,
FVII-Ile42 - sTF-Pro206,
FVII-Ser43 - sTF-Pro206,
FVII-Tyr44 - sTF-Pro206,
FVII-Leu39 - sTF-Val207,
FVII-Phe40 - sTF-Val207, and
FVII-Ser43 - sTF-Val207,
in particular at least one of the amino acids sets
FVII-Phe40 - sTF-Val207,
FVII-Ser43 - sTF-Pro206,
FVII-Gln64 - sTF-Gly109,
FVII-Glu77 - sTF-Asp58, and
FVII-Phe275 - sTF-Trp45,
have been replaced by an amino acid with a reactive side chain which gives rise to said direct bond(s).

**9.** The complex according to any one of the preceding claims, wherein at least one direct bond is between two cysteine amino acids.

**10.** The complex according to any one of the preceding claims, wherein at least one direct bond is formed via a photo-reactive amino acid.

**11.** The complex according to claim 10, wherein said photo-reactive amino acid is selected from the group consisting of photo-Ile, photo-Leu and photo-Met.

**12.** A covalent complex of a functionally active Factor VII polypeptide and a Tissue Factor polypeptide, wherein said Factor VII polypeptide and said Tissue Factor polypeptide are covalently linked by means of a linker moiety between amino acid side chains of one or more sets of (i) an amino acid of the Factor VII polypeptide and (ii) an amino acid of the Tissue Factor polypeptide, wherein said linker moiety is derived from a heterobifunctional reagent.

**13.** A method for production of a complex of a functionally active Factor VII polypeptide and a Tissue Factor polypeptide as defined in any one of the claims 1-12, said method comprising a) transfecting a cell with (i) an expression vector comprising a nucleic acid molecule encoding the Factor VII polypeptide and expression control regions operatively linked to thereto and (ii) and an expression vector comprising a nucleic acid molecule encoding the Tissue Factor polypeptide and expression control regions operatively linked to thereto, b) culturing the transfected cell under conditions for expression of Factor VII polypeptide and Tissue Factor polypeptide, and c) isolating the expressed complex by suitable means.

**Patentansprüche**

**1.** Kovalenter Komplex aus einem funktionell aktiven Faktor-VII-Polypeptid und einem Gewebefaktorpolypeptid, wobei das Faktor-VII-Polypeptid und das Gewebefaktorpolypeptid mithilfe von einer oder mehreren direkten Bindungen zwischen Aminosäureseitenketten von Sets (i) einer Aminosäure des Faktor-VII-Polypeptids und (ii) einer Aminosäure des Gewebefaktorpolypeptids kovalent verbunden sind.

2. Komplex nach Anspruch 1, wobei die wie durch den *in-vitro*-Proteolyseassay bestimmte proteolytische Aktivität des Faktor-VII-Polypeptids mindestens das 2-Fache, wie mindestens das 50-Fache, z.B. mindestens das 100-Fache, wie mindestens das 200-Fache oder mindestens das 250-Fache derjenigen des freien nativen (vom Wildtyp) Faktors VIIa beträgt.

3. Komplex nach einem der vorangehenden Ansprüche, wobei die Sets von Aminosäuren eine oder mehrere Cysteinaminosäuren umfassen.

4. Komplex nach Anspruch 3, wobei jedes der Sets eine Cysteinaminosäure umfasst.

5. Komplex nach Anspruch 3, wobei jedes der Sets zwei Cysteinaminosäuren umfasst.

6. Komplex nach einem der vorangehenden Ansprüche, wobei mindestens eine der Aminosäuren in den Positionen Thr17, Lys20, Ile22, Ser42, Gly43, Asp44, Trp45, Lys46, Ser47, Phe50, Cys57, Asp58, Asp61, Gly109, Gln110, Leu133, Ser205, Pro206 und Val207 des löslichen Gewebefaktorpolypeptids, insbesondere Trp45, Asp58, Gly109, Pro206 und Val207 des löslichen Gewebefaktorpolypeptids durch eine Aminosäure mit einer reaktiven Seitenkette ersetzt worden ist, was zu der (den) direkten Bindung(en) führt.

7. Komplex nach einem der vorangehenden Ansprüche, wobei mindestens eine der Aminosäuren in den Positionen Leu39, Phe40, Ile42, Ser43, Tyr44, Gln64, Leu65, Ile69, Glu77, Gly78, Arg79, Va192, Phe275, Val276, Arg277, Met306, Thr307 und Glu325 des Faktor-VII-Polypeptids, insbesondere Phe40, Ser43, Gln64 Glu77 und Phe275 des Faktor-VII-Polypeptids durch eine Aminosäure mit einer reaktiven Seitenkette ersetzt worden ist, was zu der (den) direkten Bindung(en) führt.

8. Komplex nach einem der vorangehenden Ansprüche, wobei eine oder beide der Aminosäuren von mindestens einem der Aminosäuresets
FVII-Ile69 - sTF-Thr17,
FVII-Ile69 - sTF-Lys20,
FVII-Arg79 - sTF-Ile22,
FVII-Val92 - sTF-Ser47,
FVII-Val92 - sTF-Phe50,
FVII-Gly78 - sTF-Cys57,
FVII-Glu77 - sTF-Asp58,
FVII-Gly78 - sTF-Asp58,
FVII-Arg79 - sTF-Asp58,
FVII-Arg277 - sTF-Ser42,
FVII-Val276 - sTF-Gly43,
FVII-Arg277 - sTF-Gly43,
FVII-Phe275 - sTF-Asp44,
FVII-Val276 - sTF-Asp44,
FVII-Arg277 - sTF-Asp44,
FVII-Phe275 - sTF-Trp45,
FVII-Val276 - sTF-Trp45,
FVII-Argl34 - sTF-Trp45,
FVII-Phe275 - sTF-Lys46,
FVII-Gln64 - sTF-Gly109,
FVII-Gln64 - sTF-Gln110,
FVII-Leu65 - sTF-Gln110,
FVII-Phe71 - sTF-Leu133,
FVII-Ser43 - sTF-Ser205,
FVII-Leu39 - sTF-Pro206,
FVII-Phe40 - sTF-Pro206,
FVII-Ile42 - sTF-Pro206,
FVII-Ser43 - sTF-Pro206,
FVII-Tyr44 - sTF-Pro206,
FVII-Leu39 - sTF-Val207,
FVII-Phe40 - sTF-Val207 und
FVII-Ser43 - sTF-Val207,

insbesondere mindestens einem der Aminosäuresets
FVII-Phe40 - sTF-Val207,
FVII-Ser43 - sTF-Pro206,
FVII-Gln64 - sTF-Gly109,
FVII-Glu77 - sTF-Asp58 und
FVII-Phe275 - sTF-Trp45,
durch eine Aminosäure mit einer reaktiven Seitenkette ersetzt worden ist, was zu der (den) direkten Bindung(en) führt.

**9.** Komplex nach einem der vorangehenden Ansprüche, wobei mindestens eine direkte Bindung zwischen zwei Cysteinaminosäuren vorliegt

**10.** Komplex nach einem der vorangehenden Ansprüche, wobei mindestens eine direkte Bindung über eine fotoreaktive Aminosäure gebildet wird.

**11.** Komplex nach Anspruch 10, wobei die fotoreaktive Aminosäure ausgewählt ist aus der Gruppe, bestehend aus Foto-Ile, Foto-Leu und Foto-Met.

**12.** Kovalenter Komplex aus einem funktionellen aktiven Faktor-VII-Polypeptid und einem Gewebefaktorpolypeptid, wobei das Faktor-VII-Polypeptid und das Gewebefaktorpolypeptid mithilfe einer Verbindungsgruppen-Einheit zwischen Aminosäureseitenketten von einem oder mehreren Sets (i) einer Aminosäure des Faktor-VII-Polypeptids und (ii) einer Aminosäure des Gewebefaktorpolypeptids kovalent verbunden sind, wobei die Verbindungsgruppen-Einheit von einem heterobifunktionellen Reagens abgeleitet ist.

**13.** Verfahren zur Herstellung eines wie in einem der Ansprüche 1-12 definierten Komplexes aus einem funktionell aktiven Faktor-VII-Polypeptid und einem Gewebefaktorpolypeptid, wobei das Verfahren a) Transfizieren einer Zelle mit (i) einem Expressionsvektor, der ein das Faktor-VII-Polypeptid kodierendes Nukleinsäuremolekül und daran funktionsfähig gebundene Expressionssteuerregionen umfasst, und (ii) einem Expressionsvektor, der ein das Gewebefaktorpolypeptid kodierendes Nukleinsäuremolekül und daran funktionsfähig gebundene Expressionssteuerregionen umfasst, b) Kultivieren der transfizierten Zelle unter Bedingungen zur Expression von Faktor-VII-Polypeptid und Gewebefaktorpolypeptid und c) Isolieren des exprimierten Komplexes durch geeignete Mittel umfasst.

## Revendications

**1.** Complexe covalent d'un polypeptide Facteur VII fonctionnellement actif et d'un polypeptide Facteur Tissulaire, dans lequel ledit polypeptide Facteur VII et ledit polypeptide Facteur Tissulaire sont liés par liaison covalente par l'intermédiaire d'une ou plusieurs liaisons directes entre des chaînes latérales d'acides aminés d'ensembles (i) d'un acide aminé du polypeptide Facteur VII et (ii) d'un acide aminé du polypeptide Facteur Tissulaire.

**2.** Complexe selon la revendication 1, dans lequel l'activité protéolytique du polypeptide Facteur VII est au moins 2 fois, par exemple au moins 50 fois, p.ex. au moins 100 fois, notamment au moins 200 fois, ou au moins 250 fois supérieure à celle du Facteur VIIa natif libre (de type sauvage), telle que déterminée par un essai de protéolyse in vitro.

**3.** Complexe selon l'une quelconque des revendications précédentes, dans lequel les ensembles des acides aminés comprennent un ou plusieurs acides aminés cystéines.

**4.** Complexe selon la revendication 3, dans lequel chacun des ensembles comprend un acide aminé cystéine.

**5.** Complexe selon la revendication 3, dans lequel chacun des ensembles comprend deux acides aminés cystéines.

**6.** Complexe selon l'une quelconque des revendications précédentes, dans lequel au moins l'un des acides aminés sur les positions Thr17, Lys20, Ile22, Ser42, Gly43, Asp44, Trp45, Lys46, Ser47, Phe50, Cys57, Asp58, Asp61, Gly109, Gln110, Leu133, Ser205, Pro206 et Val207 du polypeptide Facteur Tissulaire soluble, en particulier Trp45, Asp58, Gly109, Pro206 et Val207 du polypeptide Facteur Tissulaire soluble a été remplacé par un acide aminé ayant une chaîne latérale réactive qui donne naissance à ladite ou auxdites liaisons directes.

**7.** Complexe selon l'une quelconque des revendications précédentes, dans lequel au moins l'un des acides aminés sur les positions Leu39, Phe40, Ile42, Ser43, Tyr44, Gln64, Leu65, Ile69, Glu77, Gly78, Arg79, Val92, Phe275,

Val276, Arg277, Met306, Thr307, et Lu325 du polypeptide Facteur VII, en particulier Phe40, Ser43, Gln64 Lu77 et Phe275 du polypeptide Facteur VII a été remplacé par un acide aminé ayant une chaîne latérale réactive qui donne naissance à ladite ou auxdites liaisons directes.

8. Complexe selon l'une quelconque des revendications précédentes, dans lequel l'un des acides aminés, ou les deux, d'au moins l'un des ensembles d'acides aminés
FVII-Ile69 - sTF-Thr17,
FVII-Ile69 - sTF-Lys20,
FVII-Arg79 - sTF-Ile22,
FVII-Val92 - sTF-Ser47,
FVII-Val92 - sTF-Phe50,
FVII-Gly78 - sTF-Cys57,
FVII-Glu77 - sTF-Asp58,
FVII-Gly78 - sTF-Asp58,
FVII-Arg79 - sTF-Asp58,
FVII-Arg277 - sTF-Ser42,
FVII-Val276 - sTF-Gly43,
FVII-Arg277 - sTF-Gly43,
FVII-Phe275 - sTF-Asp44,
FVII-Va1276 - sTF-Asp44,
FVII-Arg277 - sTF-Asp44,
FVII-Phe275 - sTF-Trp45,
FVII-Val276 - sTF-Trp45,
FVII-Argl34 - sTF-Trp45,
FVII-Phe275 - sTF-Lys46,
FVII-Gln64 - sTF-Gly109,
FVII-Gln64 - sTF-Gln110,
FVII-Leu65 - sTF-Gln110,
FVII-Phe71 - sTF-Leu133,
FVII-Ser43 - sTF-Ser205,
FVII-Leu39 - sTF-Pro206,
FVII-Phe40 - sTF-Pro206,
FVII-Ile42 - sTF-Pro206,
FVII-Ser43 - sTF-Pro206,
FVII-Tyr44 - sTF-Pro206,
FVII-Leu39 - sTF-Val207,
FVII-Phe40 - sTF-Val207, et
FVII-Ser43 - sTF-Val207,
en particulier de l'un des ensembles d'acides aminés
FVII-Phe40 - sTF-Val207,
FVII-Ser43 - sTF-Pro206,
FVII-Gln64 - sTF-Gly109,
FVII-Glu77 - sTF-Asp58, et
FVII-Phe275 - sTF-Trp45,
ont été remplacés par un acide aminé ayant une chaîne latérale réactive qui donne naissance à ladite ou auxdites liaisons directes.

9. Complexe selon l'une quelconque des revendications précédentes, dans lequel au moins une liaison directe se trouve entre deux acides aminés cystéines.

10. Complexe selon l'une quelconque des revendications précédentes, dans lequel au moins une liaison directe est formée par l'intermédiaire de l'acide aminé photoréactif.

11. Complexe selon la revendication 10, dans lequel ledit acide aminé photoréactif est choisi dans le groupe consistant en photo-Ile, photo-Leu et photo-Met.

12. Complexe covalent d'un polypeptide Facteur VII fonctionnellement actif et d'un polypeptide Facteur Tissulaire, dans lequel ledit polypeptide Facteur VII et ledit polypeptide Facteur Tissulaire sont liés par liaison covalente par l'intermédiaire d'un fragment lieur entre les chaînes latérales des acides aminés d'un ou plusieurs ensembles (i) d'un acide aminé du polypeptide Facteur VII et (ii) d'un acide aminé du polypeptide Facteur Tissulaire, ledit fragment lieur dérivant d'un réactif hétérobifonctionnel.

**13.** Procédé de production d'un complexe d'un polypeptide Facteur VII fonctionnellement actif et d'un polypeptide Facteur Tissulaire tel que défini dans l'une quelconque des revendications 1-12, ledit procédé comprenant a) la transfection d'une cellule avec (i) un vecteur d'expression comprenant une molécule d'acide nucléique codant pour le polypeptide Facteur VII et des régions régulatrices d'expression qui y sont liées d'une manière opérationnelle et (ii) un vecteur d'expression comprenant une molécule d'acide nucléique codant pour le polypeptide Facteur Tissulaire et des régions régulatrices d'expression qui y sont liées d'une manière opérationnelle, b) la culture de la cellule transfectée dans des conditions permettant l'expression du polypeptide Facteur VII et du polypeptide Facteur Tissulaire, et c) l'isolement du complexe exprimé, par des moyens appropriés.

**Figure 1**

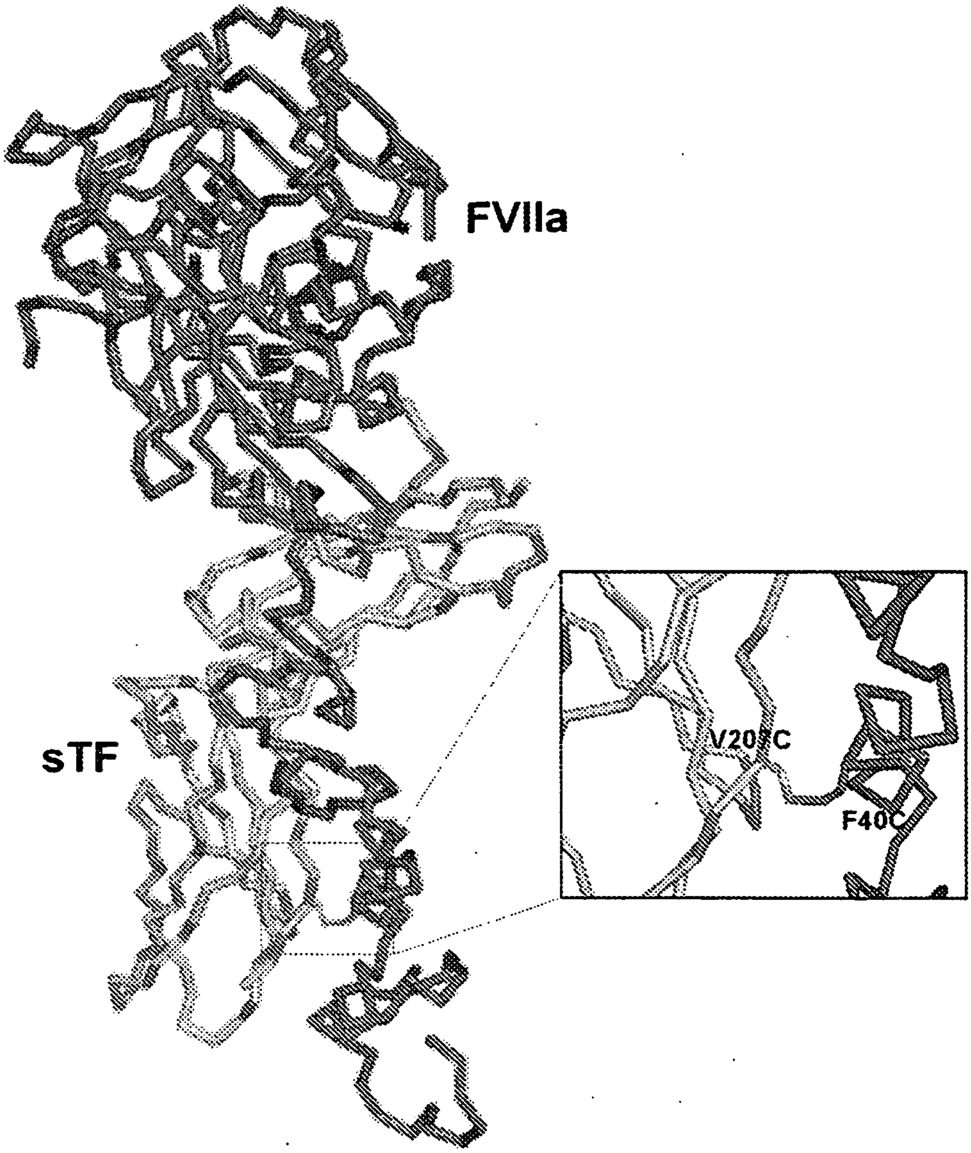

**Figure 2**

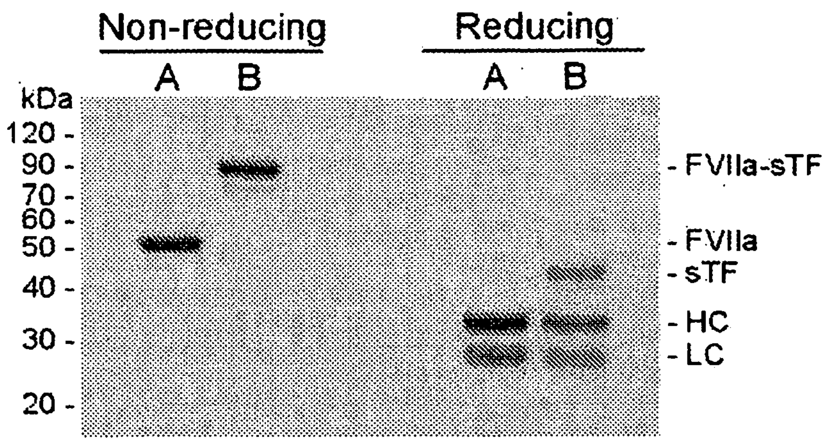


**Figure 3**

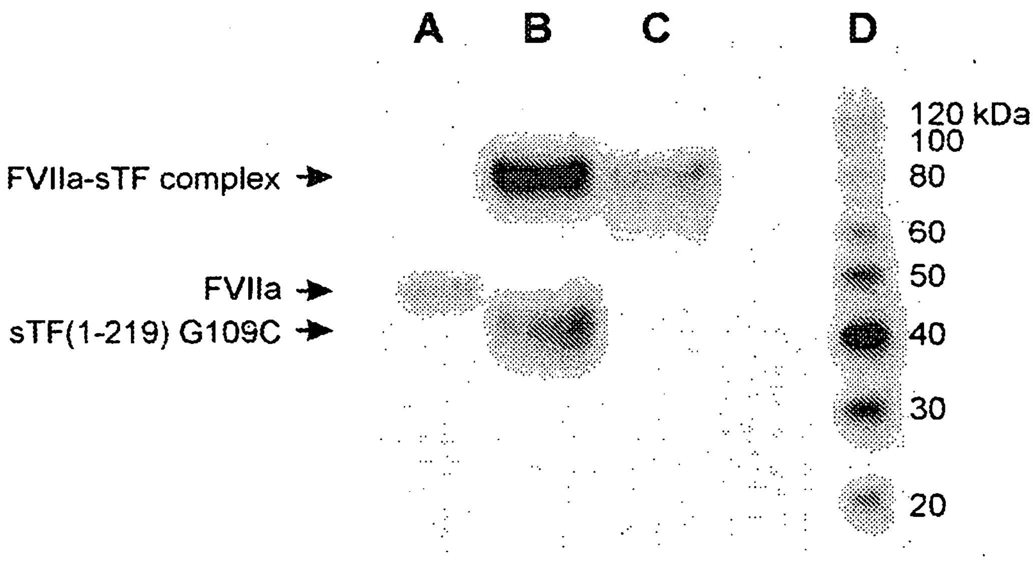

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4784950 A
- WO 0331464 A
- US 20040043446 A
- US 20040063911 A
- US 20040142856 A
- US 20040137557 A
- US 20040132640 A
- WO 0104287 A
- US 20030165996 A
- WO 0158935 A
- WO 0393465 A
- WO 0202764 A
- US 20030211094 A
- US 5580560 A
- WO 02077218 A
- WO 0238162 A
- WO 9920767 A
- US 6017882 A
- US 6747003 B
- US 20030100506 A
- WO 0066753 A
- US 20010018414 A
- US 2004220106 A
- US 200131005 B
- US 6762286 B
- US 6693075 B
- US 6806063 B
- US 20030096338 A
- WO 04029091 A
- WO 04083361 A
- WO 04111242 A
- WO 04108763 A
- WO 0183725 A
- WO 0222776 A
- WO 03027147 A
- WO 04029090 A
- JP 2001061479 B
- US 5077214 A
- WO 05075635 A


### Non-patent literature cited in the description


- **Miyata et al.** *Biochemistry,* 1997, vol. 36, 5120-5127
- **Lino et al.** *Arch. Biochem. Biophys.,* 1998, vol. 352, 182-192
- **Mollerup et al.** *Biotechnol. Bioeng.,* 1995, vol. 48, 501-505
- **Kornfelt et al.** *Arch. Biochem. Biophys.,* 1999, vol. 363, 43-54
- **Spicer et al.** *Proc. Natl. Acad. Sci. USA,* 1987, vol. 84, 5148-5152
- **Banner et al.** *Nature,* 1996, vol. 380, 41-46
- **Petersen et al.** *Protein Eng.,* 1999, vol. 12, 535-548
- **Suchanek et al.** *Nature methods,* 2005, vol. 2, 261-267
- **Zhang et al.** *Biochem. Biophys. Res. Comm.,* 1995, vol. 217 (3), 1177-1184
- **Stone et al.** *Biochem. J.,* 1995, vol. 310, 605-614
- **Freskgård et al.** *Protein Sci.,* 1996, vol. 5, 1521-1540
- **Owenius et al.** *Biophys. J.,* 1999, vol. 77, 2237-2250
- **Österlund et al.** *Biochemistry,* 2001, vol. 40, 9324-9328
- **Zhang et al.** *Biochem. Biophys. Res. Commun.,* 1995, vol. 217, 1177-1184
- **Graham et al.** *J. Gen. Virol.,* 1977, vol. 36, 59-72
- **Waechter ; Baserga.** *Proc. Narl. Acad. Sci. USA,* 1982, vol. 79, 1106-1110
- **Urlaub ; Chasin.** *Proc. Natl. Acad. Sci. USA,* 1980, vol. 77, 4216-4220
- *Cell,* 1983, vol. 33, 405
- *Somatic Cell and Molecular Genetics,* 1986, vol. 12, 555
- **Freskgård et al.** *Protein Sci.,* 1996, vol. 5, 1531-1540
- **Thim et al.** *Biochemistry,* 1988, vol. 27, 7785-7793
- **Persson et al.** *FEBS Lett.,* 1996, vol. 385, 241-243
- **Bock P.E.** *J.Biol.Chem.,* 1992, vol. 267, 14963-14973
- **Ruf et al.** *Thromb. Haemost.,* 1991, vol. 66, 529-533

SEQUENCE LISTING

<110> Novo Nordisk Health Care AG

<120> COVALENT FACTOR VII-TISSUE FACTOR COMPLEX

<130> 7275.205-EP

<160> 21

<170> PatentIn version 3.3

<210> 1
<211> 406
<212> PRT
<213> Homo sapiens

<220>
<221> MISC_FEATURE
<222> (1)..(406)
<223> Xaa means 4-carboxyglutamic acid (gamma-carboxyglutamate)

<400> 1

```
Ala Asn Ala Phe Leu Xaa Xaa Leu Arg Pro Gly Ser Leu Xaa Arg Xaa
1               5                   10                  15

Cys Lys Xaa Xaa Gln Cys Ser Phe Xaa Xaa Ala Arg Xaa Ile Phe Lys
            20                  25                  30

Asp Ala Xaa Arg Thr Lys Leu Phe Trp Ile Ser Tyr Ser Asp Gly Asp
        35                  40                  45

Gln Cys Ala Ser Ser Pro Cys Gln Asn Gly Gly Ser Cys Lys Asp Gln
    50                  55                  60

Leu Gln Ser Tyr Ile Cys Phe Cys Leu Pro Ala Phe Glu Gly Arg Asn
65                  70                  75                  80

Cys Glu Thr His Lys Asp Asp Gln Leu Ile Cys Val Asn Glu Asn Gly
                85                  90                  95

Gly Cys Glu Gln Tyr Cys Ser Asp His Thr Gly Thr Lys Arg Ser Cys
            100                 105                 110

Arg Cys His Glu Gly Tyr Ser Leu Leu Ala Asp Gly Val Ser Cys Thr
        115                 120                 125
```

Pro Thr Val Glu Tyr Pro Cys Gly Lys Ile Pro Ile Leu Glu Lys Arg
130 135 140

Asn Ala Ser Lys Pro Gln Gly Arg Ile Val Gly Gly Lys Val Cys Pro
145 150 155 160

Lys Gly Glu Cys Pro Trp Gln Val Leu Leu Leu Val Asn Gly Ala Gln
165 170 175

Leu Cys Gly Gly Thr Leu Ile Asn Thr Ile Trp Val Val Ser Ala Ala
180 185 190

His Cys Phe Asp Lys Ile Lys Asn Trp Arg Asn Leu Ile Ala Val Leu
195 200 205

Gly Glu His Asp Leu Ser Glu His Asp Gly Asp Glu Gln Ser Arg Arg
210 215 220

Val Ala Gln Val Ile Ile Pro Ser Thr Tyr Val Pro Gly Thr Thr Asn
225 230 235 240

His Asp Ile Ala Leu Leu Arg Leu His Gln Pro Val Val Leu Thr Asp
245 250 255

His Val Val Pro Leu Cys Leu Pro Glu Arg Thr Phe Ser Glu Arg Thr
260 265 270

Leu Ala Phe Val Arg Phe Ser Leu Val Ser Gly Trp Gly Gln Leu Leu
275 280 285

Asp Arg Gly Ala Thr Ala Leu Glu Leu Met Val Leu Asn Val Pro Arg
290 295 300

Leu Met Thr Gln Asp Cys Leu Gln Gln Ser Arg Lys Val Gly Asp Ser
305 310 315 320

Pro Asn Ile Thr Glu Tyr Met Phe Cys Ala Gly Tyr Ser Asp Gly Ser
325 330 335

Lys Asp Ser Cys Lys Gly Asp Ser Gly Gly Pro His Ala Thr His Tyr
340 345 350

Arg Gly Thr Trp Tyr Leu Thr Gly Ile Val Ser Trp Gly Gln Gly Cys

```
                355                 360                 365

  Ala Thr Val Gly His Phe Gly Val Tyr Thr Arg Val Ser Gln Tyr Ile
      370                 375                 380

  Glu Trp Leu Gln Lys Leu Met Arg Ser Glu Pro Arg Pro Gly Val Leu
  385                 390                 395                 400

  Leu Arg Ala Pro Phe Pro
                  405
```

<210> 2
<211> 263
<212> PRT
<213> Human

<400> 2

```
Ser Gly Thr Thr Asn Thr Val Ala Ala Tyr Asn Leu Thr Trp Lys Ser
1               5                   10                  15

Thr Asn Phe Lys Thr Ile Leu Glu Trp Glu Pro Lys Pro Val Asn Gln
            20                  25                  30

Val Tyr Thr Val Gln Ile Ser Thr Lys Ser Gly Asp Trp Lys Ser Lys
        35                  40                  45

Cys Phe Tyr Thr Thr Asp Thr Glu Cys Asp Leu Thr Asp Glu Ile Val
    50                  55                  60

Lys Asp Val Lys Gln Thr Tyr Leu Ala Arg Val Phe Ser Tyr Pro Ala
65                  70                  75                  80

Gly Asn Val Glu Ser Thr Gly Ser Ala Gly Glu Pro Leu Tyr Glu Asn
                85                  90                  95

Ser Pro Glu Phe Thr Pro Tyr Leu Glu Thr Asn Leu Gly Gln Pro Thr
            100                 105                 110

Ile Gln Ser Phe Glu Gln Val Gly Thr Lys Val Asn Val Thr Val Glu
        115                 120                 125

Asp Glu Arg Thr Leu Val Arg Arg Asn Asn Thr Phe Leu Ser Leu Arg
    130                 135                 140
```

```
Asp Val Phe Gly Lys Asp Leu Ile Tyr Thr Leu Tyr Tyr Trp Lys Ser
145                 150                 155                 160

Ser Ser Ser Gly Lys Lys Thr Ala Lys Thr Asn Thr Asn Glu Phe Leu
                165                 170                 175

Ile Asp Val Asp Lys Gly Glu Asn Tyr Cys Phe Ser Val Gln Ala Val
            180                 185                 190

Ile Pro Ser Arg Thr Val Asn Arg Lys Ser Thr Asp Ser Pro Val Glu
        195                 200                 205

Cys Met Gly Gln Glu Lys Gly Glu Phe Arg Glu Ile Phe Tyr Ile Ile
    210                 215                 220

Gly Ala Val Val Phe Val Val Ile Ile Leu Val Ile Ile Leu Ala Ile
225                 230                 235                 240

Ser Leu His Lys Cys Arg Lys Ala Gly Val Gly Gln Ser Trp Lys Glu
                245                 250                 255

Asn Ser Pro Leu Asn Val Ser
            260
```

<210> 3
<211> 1335
<212> DNA
<213> Human

<400> 3

```
atggtctccc aggccctcag gctcctctgc cttctgcttg ggcttcaggg ctgcctggct      60
gcagtcttcg taacccagga ggaagcccaa ggcgtcctgc accggcgccg gcgcgccaac     120
gcgttcctgg aggagctgcg gccgggctcc ctggagaggg agtgcaagga ggagcagtgc     180
tccttcgagg aggcccggga gatcttcaag gacgcggaga ggacgaagct gttctggatt     240
tcttacagtg atggggacca gtgtgcctca agtccatgcc agaatggggg ctcctgcaag     300
gaccagctcc agtcctatat ctgcttctgc ctccctgcct tcgagggccg gaactgtgag     360
acgcacaagg atgaccagct gatctgtgtg aacgagaacg gcggctgtga gcagtactgc     420
agtgaccaca cgggcaccaa gcgctcctgt cggtgccacg aggggtactc tctgctggca     480
gacggggtgt cctgcacacc cacagttgaa tatccatgtg gaaaaatacc tattctagaa     540
```

```
aaaagaaatg ccagcaaacc ccaaggccga attgtggggg gcaaggtgtg ccccaaaggg    600
gagtgtccat ggcaggtcct gttgttggtg aatggagctc agttgtgtgg ggggaccctg    660
atcaacacca tctgggtggt ctccgcggcc cactgtttcg acaaaatcaa gaactggagg    720
aacctgatcg cggtgctggg cgagcacgac ctcagcgagc acgacgggga tgagcagagc    780
cggcgggtgg cgcaggtcat catccccagc acgtacgtcc cgggcaccac caaccacgac    840
atcgcgctgc tccgcctgca ccagcccgtg gtcctcactg accatgtggt gcccctctgc    900
ctgcccgaac ggacgttctc tgagaggacg ctggccttcg tgcgcttctc attggtcagc    960
ggctggggcc agctgctgga ccgtggcgcc acggccctgg agctcatggt cctcaacgtg   1020
ccccggctga tgacccagga ctgcctgcag cagtcacgga aggtgggaga ctccccaaat   1080
atcacggagt acatgttctg tgccggctac tcggatggca gcaaggactc ctgcaagggg   1140
gacagtggag gcccacatgc cacccactac cggggcacgt ggtacctgac gggcatcgtc   1200
agctggggcc agggctgcgc aaccgtgggc cactttgggg tgtacaccag ggtctcccag   1260
tacatcgagt ggctgcaaaa gctcatgcgc tcagagccac gcccaggagt cctcctgcga   1320
gccccatttc cctag                                                    1335
```

<210> 4
<211> 219
<212> PRT
<213> Human

<400> 4

```
Ser Gly Thr Thr Asn Thr Val Ala Ala Tyr Asn Leu Thr Trp Lys Ser
1               5                   10                  15

Thr Asn Phe Lys Thr Ile Leu Glu Trp Glu Pro Lys Pro Val Asn Gln
            20                  25                  30

Val Tyr Thr Val Gln Ile Ser Thr Lys Ser Gly Asp Trp Lys Ser Lys
        35                  40                  45

Cys Phe Tyr Thr Thr Asp Thr Glu Cys Asp Leu Thr Asp Glu Ile Val
    50                  55                  60

Lys Asp Val Lys Gln Thr Tyr Leu Ala Arg Val Phe Ser Tyr Pro Ala
65                  70                  75                  80

Gly Asn Val Glu Ser Thr Gly Ser Ala Gly Glu Pro Leu Tyr Glu Asn
```

```
                85                  90                  95

Ser Pro Glu Phe Thr Pro Tyr Leu Glu Thr Asn Leu Gly Gln Pro Thr
            100                 105                 110

Ile Gln Ser Phe Glu Gln Val Gly Thr Lys Val Asn Val Thr Val Glu
        115                 120                 125

Asp Glu Arg Thr Leu Val Arg Arg Asn Asn Thr Phe Leu Ser Leu Arg
    130                 135                 140

Asp Val Phe Gly Lys Asp Leu Ile Tyr Thr Leu Tyr Tyr Trp Lys Ser
145                 150                 155                 160

Ser Ser Ser Gly Lys Lys Thr Ala Lys Thr Asn Thr Asn Glu Phe Leu
                165                 170                 175

Ile Asp Val Asp Lys Gly Glu Asn Tyr Cys Phe Ser Val Gln Ala Val
            180                 185                 190

Ile Pro Ser Arg Thr Val Asn Arg Lys Ser Thr Asp Ser Pro Val Glu
        195                 200                 205

Cys Met Gly Gln Glu Lys Gly Glu Phe Arg Glu
    210                 215
```

<210> 5
<211> 756
<212> DNA
<213> human

<400> 5

```
atggagaccc ctgcctggcc ccgggtcccg cgccccgaga ccgccgtcgc tcggacgctc     60

ctgctcggct gggtcttcgc ccaggtggcc ggcgcttcag gcactacaaa tactgtggca    120

gcatataatt taacttggaa atcaactaat ttcaagacaa ttttggagtg ggaacccaaa    180

cccgtcaatc aagtctacac tgttcaaata agcactaagt caggagattg gaaaagcaaa    240

tgcttttaca caacagacac agagtgtgac ctcaccgacg agattgtgaa ggatgtgaag    300

cagacgtact tggcacgggt cttctcctac ccggcaggga atgtggagag caccggttct    360

gctggggagc ctctgtatga gaactcccca gagttcacac cttacctgga gacaaacctc    420

ggacagccaa caattcagag ttttgaacag gtgggaacaa aagtgaatgt gaccgtagaa    480
```

gatgaacgga ctttagtcag aaggaacaac actttcctaa gcctccggga tgtttttggc 540
aaggacttaa tttatacact ttattattgg aaatcttcaa gttcaggaaa gaaaacagcc 600
aaaacaaaca ctaatgagtt tttgattgat gtggataaag gagaaaacta ctgtttcagt 660
gttcaagcag tgattccctc ccgaacagtt aaccggaaga gtacagacag cccggtagag 720
tgtatgggcc aggagaaagg ggaattcaga gaataa 756

<210> 6
<211> 44
<212> DNA
<213> Artificial

<220>
<223> DNA primer

<400> 6
ggcggcggct agcatggtct cccaggccct caggctcctc tgcc 44

<210> 7
<211> 41
<212> DNA
<213> Artificial

<220>
<223> DNA primer

<400> 7
ccgccgccgc ggccgcctag ggaaatgggg ctcgcaggag g 41

<210> 8
<211> 40
<212> DNA
<213> Artificial

<220>
<223> DNA primer

<400> 8
gcggagagga cgaagctgtg ctggatttct tacagtgatg 40

<210> 9
<211> 40
<212> DNA
<213> Artificial

<220>
<223> DNA primer

<400> 9
catcactgta agaaatccag cacagcttcg tcctctccgc 40

<210> 10

<211> 38

<212> DNA
<213> Artificial

<220>
<223> DNA primer

<400> 10
ggcggcgggc tagcatggag acccctgcct ggccccgg 38

<210> 11
<211> 42
<212> DNA
<213> Artificial

<220>
<223> DNA primer

<400> 11
ccgccgccct cgagttattc tctgaattcc cctttctcct gg 42

<210> 12
<211> 34
<212> DNA
<213> Artificial

<220>
<223> DNA primer

<400> 12
gagtacagac agcccgtgcg agtgtatggg ccag 34

<210> 13
<211> 34
<212> DNA
<213> Artificial

<220>
<223> DNA primer <400> 13
ctggcccata cactcgcacg ggctgtctgt actc 34

<210> 14
<211> 37
<212> DNA
<213> Artificial

<220>
<223> DNA primer

<400> 14
cactaagtca ggagattgca aaagcaaatg cttttac 37

<210> 15
<211> 37
<212> DNA
<213> Artificial

<220>
<223> DNA primer

<400> 15
gtaaaagcat ttgcttttgc aatctcctga cttagtg 37

<210> 16
<211> 36
<212> DNA
<213> Artificial

<220>
<223> DNA primer

<400> 16
caacagacac agagtgttgc ctcaccgacg agattg 36

<210> 17
<211> 36
<212> DNA
<213> Artificial

<220>
<223> DNA primer

<400> 17
caatctcgtc ggtgaggcaa cactctgtgt ctgttg 36

<210> 18
<211> 36
<212> DNA
<213> Artificial

<220>
<223> DNA primer

<400> 18
cctggagaca aacctctgcc agccaacaat tcagag 36

<210> 19
<211> 36
<212> DNA
<213> Artificial

<220>
<223> DNA primer

<400> 19
ctctgaattg ttggctggca gaggtttgtc tccagg 36

<210> 20
<211> 34
<212> DNA
<213> Artificial

<220>
<223> DNA primer

<400> 20
gaagagtaca gacagctgcg tagagtgtat gggc 34

<210> 21
<211> 34
<212> DNA
<213> Artificial

<220>
<223> DNA primer

<400> 21
gcccatacac tctacgcagc tgtctgtact cttc 34